# EUROPÄISCHE PATENTSCHRIFT

(11) **EP 2 771 485 B1**
(45) Veröffentlichungstag und Bekanntmachung des Hinweises auf die Patenterteilung: **11.07.2018**
(21) Anmeldenummer: 12780712.1
(22) Anmeldetag: 25.10.2012
(51) Int. Cl.: C12Q 1/6811, C12N 15/10

(54) **VERFAHREN ZUR IDENTIFIKATION VON APTAMEREN**
PROCEDURE FOR THE IDENTIFICATION OF APTAMERS
PROCEDE POUR IDENTIFIER DES APTAMÈRES

(30) Priorität: 28.10.2011 DE 102011085473
(43) Veröffentlichungstag der Anmeldung: 03.09.2014
(73) Patentinhaber: Albert-Ludwigs-Universität Freiburg, 79085 Freiburg (DE)
(72) Erfinder: ROTH, Günther, 79110 Freiburg im Breisgau (DE); REINEMANN, Christine, 04275 Leipzig (DE); STREHLITZ, Beate, 04316 Leipzig (DE)
(74) Vertreter: Hertin und Partner Rechts- und Patentanwälte PartG mbB
(86) Internationale Anmeldenummer: PCT/EP2012/071155
(87) Internationale Veröffentlichungsnummer: WO 2013/060777

(56) Entgegenhaltungen:
- WO-A1-2010/100265
- WO-A2-2008/022332
- WO-A2-2009/151688
- US-A1- 2010 055 677
- US-A1- 2011 262 922
- M. CHO ET AL: "Quantitative selection of DNA aptamers through microfluidic selection and high-throughput sequencing", PROCEEDINGS OF THE NATIONAL ACADEMY OF SCIENCES, Bd. 107, Nr. 35, 31. August 2010 (2010-08-31), Seiten 15373-15378, XP055058044, ISSN: 0027-8424, DOI: 10.1073/pnas.1009331107
- PENG LU ET AL: "A combined atomic force/fluorescence microscopy technique to select aptamers in a single cycle from a small pool of random oligonucleotides.", MICROSCOPY RESEARCH AND TECHNIQUE APR 2007, Bd. 70, Nr. 4, April 2007 (2007-04), Seiten 372-381, XP002694714, ISSN: 1059-910X
- MARK PLATT ET AL: "Analysis of aptamer sequence activity relationships", INTEGRATIVE BIOLOGY, Bd. 1, Nr. 1, 1. Januar 2009 (2009-01-01), Seite 116, XP055058069, ISSN: 1757-9694, DOI: 10.1039/b814892a
- KEKE SHAO ET AL: "Emulsion PCR: A High Efficient Way of PCR Amplification of Random DNA Libraries in Aptamer Selection", PLOS ONE, Bd. 6, Nr. 9, 1. Januar 2011 (2011-01-01), Seite e24910, XP055058036, ISSN: 1932-6203, DOI: 10.1371/journal.pone.0024910

## Beschreibung

Die vorliegende Erfindung betrifft ein Verfahren zur Identifikation von Aptameren. Aptamere sind künstliche Oligonukleotide, die aus einer Oligonukleotidbibliothek hinsichtlich ihrer Bindungseigenschaften gegenüber einer gewünschten Zielstruktur, auch bezeichnet als Target, selektiert wurden. Die Entwicklung von DNA Aptameren erfolgt bisher nach einem in vitro Selektionsverfahren, wobei aus einer komplexen Bibliothek von ca. 10¹⁵ verschiedenen Molekülen diejenigen mit den besten Bindungseigenschaften zum Target selektiert werden. Die Methode heißt SELEX (Systematic Evolution of Ligands by Exponential Enrichment), und die Grundlagen dafür wurden 1990 beschrieben (Ellington, A. D., Szostak, J. W., Nature 346 (1990) 818-822, Tuerk, C., Gold, L., Science 249 (1990) 505-510, Robertson, D. L., Joyce, G. F., Nature 344 (1990) 467-468). Der SELEX Prozess wird in vielen Variationen für die Aptamer-Selektion angewendet (Stoltenburg, R., Reinemann, C., Strehlitz, B., Biomol. Eng. 24 (2007) 381-403)).
Der SELEX-Prozess zur Gewinnung von DNA Aptameren ist ein iterativer Prozess. Ausgangspunkt ist eine chemisch synthetisierte DNA Bibliothek mit ca. 10¹⁵ unterschiedlichen Sequenzen. Die einzelnen DNA-Moleküle der Bibliothek verfügen dabei über einen randomisierten Sequenz-Bereich von z.B. 20 bis 80 Nukleotiden Länge, flankiert von jeweils spezifischen Primer-Sequenzen am 3' und 5' Ende von beispielsweise 18 bis 21 Nukleotiden Länge, die in jedem Element der Bibliothek gleich sind. Ein Prozess-Zyklus, der auch als (Selektions-)Runde bezeichnet wird, besteht aus den Schritten:
∘ Bindung mit dem Target,
∘ Entfernung ungebundener DNA,
∘ Elution der gebundenen DNA vom Target,
∘ Amplifikation der vormals gebundenen DNA mittels PCR,
∘ sowie Gewinnung der relevanten ssDNA aus den PCR-Produkten.
Das Ergebnis der vorangehenden Runde ist jeweils das Ausgangsmaterial für die nachfolgende Runde. Nach ca. 6 bis 20 Runden sollten sich Sequenzmotive mit hoher Affinität und Spezifität für das Target angereichert haben. Die Triebkräfte dieses evolutiven Prozesses sind die Selektionsbedingungen, die durch die Eigenschaften des Targets, seine Konzentration, die Pufferbedingungen der Lösung, Temperatur, Inkubationszeit, Effizienz der Abtrennung ungebundener DNA, Einführung von Negativ-Selektions-Schritten u.a. bestimmt werden. Dabei hängt die Affinität und Spezifität der Aptamere von der Stringenz der Bedingungen ab, die im Verlauf der Selektions-Runden angepasst werden kann.
Die Funktionalität der Aptamere, d.h. ihre Bindungsfähigkeit an das Target, wird durch ihre dreidimensionale Struktur begründet, die von ihrer Primärsequenz, der Länge der Nukleinsäuremoleküle (vorzugsweise <100 Nukleotide) und den Umgebungsbedingungen abhängt. Aptamere bilden typische Strukturmotive wie stems, internal loops, tetra loops, triplicates, pseudoknots, hairpin structures, kissing complexes oder G-quadruplex Strukturen aus. Aptamere gehen bei Anwesenheit des Targets adaptive Konformationsänderungen ein, so dass ihre dreidimensionale Faltung eine spezifische Bindungsstelle für das Target ausbildet (induced fit). Die Bindung zwischen Aptamer und Target basiert auf verschiedenen intermolekularen Wechselwirkungen wie Strukturkompatibilität, Stapelung aromatischer Ringe mit den Nukleinsäurebasen der Aptamere, elektrostatische Wechselwirkungen zwischen geladenen Gruppen und Wasserstoffbrückenbindungen.

Aptamere wurden für sehr unterschiedliche Targets entwickelt: anorganische und kleine Moleküle, Peptide, Proteine, Kohlenhydrate, Antibiotika. Auch komplexe Targets wie ganze Zellen und Organismen sowie Target-Mischungen wurden für die Aptamerselektion eingesetzt. Auch toxische und nicht-immunogene Targets, die beispielsweise einer Antikörperherstellung nicht zugänglich sind, können verwendet werden. Nach ihrer Selektion und Sequenzanalyse erfolgt die Herstellung der Aptamere mittels chemischer Synthese, wobei die Reproduzierbarkeit gesichert und die Menge im Prinzip nicht eingeschränkt ist. Modifizierungen der Aptamere sind einfach durchzuführen, z.B. für die Immobilisierung auf Sensor- oder Chipoberflächen, für die Quantifizierung oder zur Verbesserung der Stabilität. Die Aptamerbindung ist reversibel und denaturierte Aptamere sind regenerierbar. Beides sind große Vorteile für die analytische Anwendung der Aptamere. Aptamere zeigen große Ähnlichkeit zu Antikörpern bezüglich des Bindungsverhaltens. Viele ihrer Eigenschaften machen die Aptamere zu ernsthaften oder gar überlegenen Alternativen zu Antikörpern. Dies liegt hauptsächlich in der reversiblen Denaturierung, der deutlich besseren Lagerbarkeit und der höheren chemischen Stabilität begründet.

Neben dem SELEX-Verfahren sind zwei weitere Verfahren zur Aptamerselektion bekannt. Das Monolex-Verfahren der Firma Aptares geht ebenfalls von einer Oligonukleotid-Bibliothek aus. Nach einer Affinitätsadsorption der Oligonukleotidbibliothek erfolgt eine Affinitätssortierung der Oligonukleotide an einem Adsorptionsbett und eine Auftrennung von Oligonukleotiden unterschiedlicher Affinität in verschiedene Pools. Diese Pools werden jeweils amplifiziert und es liegen dann polyklonale Aptamer-Pools von jeweils bestimmter Affinität vor. Zur Gewinnung der einzelnen (monoklonalen) Aptamere wird der jeweilige Pool polyklonaler Aptamere kloniert und sequenziert (http://www.aptares.net/html/technologie.html).
Ein Verfahren zur Herstellung von Spiegelmeren wird von der Firma NOXXON zur Entwicklung von RNA-Aptameren als Pharmazeutika verwendet. Mit diesem Verfahren werden RNA-Liganden, sogenannte Spiegelmere, entwickelt, die statt D-Ribose L-Ribose als Zuckerkomponente enthalten. Der Einbau von L-Ribose in das Zucker-Phosphat-Rückgrat der Nukleinsäure bewirkt, dass sich die dreidimensionale Struktur einer L-RNA spiegelbildlich zu der Struktur der ihr entsprechenden D-RNA verhält. L-RNA-Moleküle sind vor enzymatischer Spaltung durch Nukleasen geschützt und damit deutlich stabiler als entsprechende D-RNA. Zunächst werden Liganden zum Enantiomer der eigentlichen Zielstruktur unter Verwendung der in vitro-Evolution selektiert. Sind diese Liganden gefunden, kloniert, sequenziert und charakterisiert so wird auf chemischem Weg die entsprechende L-Ribonukleinsäure, das Spiegelmer, synthetisiert. Anschließend können durch Überprüfen der Bindungseigenschaften des Liganden gegenüber seinem eigentlichen Targetmolekül und auch gegenüber dessen Enantiomer, Aussagen über die Affinität und Spezifität des Spiegelmers getroffen werden. WO 2009/151688 offenbart die Herstellung eines speziellen Aptamergemischs und Verfahren, dieses Aptamergemisch zur Selektion guter Binder zu nutzen. In einem Verfahren ist das Aptamergemisch für den Selektionsschritt auf einem Mikrochip gebunden. Wie oben erwähnt, sollten sich in einem SELEX-Prozess nach ca. 6 bis 20 Runden Sequenzmotive mit hoher Affinität und Spezifität für das Target angereichert haben. Anschließend werden die bindenden Oligonukleotide aus dem angereicherten Oligonukleotid-Pool zur Vereinzelung kloniert und transformiert z.B. in E.coli. Danach schließen sich weitere Schritte an, wie die Überprüfung der positiven Transformanten hinsichtlich der Korrektheit der Plasmid-DNA (pDNA) und des eingebauten Inserts (PCR-Produkt) und die Präparation der pDNA.
Nachteilig bei diesem Verfahren sind der relativ hohe Zeitaufwand für das Durchlaufen der bis zu 20 SELEX-Runden und die umfangreichen Arbeiten im Anschluss an den SELEX-Prozess. Sollten während einer der bis zu 20 SELEX-Runden die Selektionsbedingungen unvorteilhaft gewählt sein, so können gute Binder verloren gehen. Nach der Klonierung erfolgt beim bisherigen Verfahren die Auswahl der weiter zu charakterisierenden Aptamere zufällig, so dass ebenfalls einzelne gute Binder verloren gehen können, weil sie zufälligerweise nicht ausgewählt, nicht weiter charakterisiert und daher nicht als Aptamere erfasst werden.
Eine Aufgabe der vorliegenden Erfindung bestand darin, ein Verfahren bereitzustellen, das diese Nachteile nicht aufweist.

Diese Aufgabe wird gelöst durch ein Verfahren zur Identifizierung und/oder Gewinnung von Aptameren, umfassend
- das in Kontakt bringen eines Gemisches von Oligonukleotiden mit einer Aptamer-Zielstruktur, und Binden zumindest eines Teils der Oligonukleotide an die Zielstruktur,- das Abtrennen der Oligonukleotide, die an die Aptamer-Zielstruktur gebunden haben, von der Aptamer-Zielstruktur und von nicht an die Aptamer-Zielstruktur angebundenen Oligonukleotiden,
- das räumlich getrennte Amplifizieren aller Oligonukleotide, die an die Aptamer-Zielstruktur gebunden haben, und das Erzeugen räumlich getrennter Amplifikate für jedes Oligonukleotid, wobei jedes Amplifikat vorwiegend eine Sorte Oligonukleotide enthält,
- das Vergeben einer spezifischen Ortskennzeichnung an jedes der räumlich getrennten Amplifikate, so dass jedes der gekennzeichneten Amplifikate anhand seiner spezifischen Ortskennzeichnung eindeutig identifizierbar ist,
- das Sequenzieren von Oligonukleotiden der gekennzeichneten Amplifikate und die Zuordnung der für das Amplifikat spezifischen Ortskennzeichnung zu der Sequenz der Sorte Oligonukleotide in dem Amplifikat,
- Herstellen eines Arrays der Amplifikate, wobei die amplifizierten Oligonukleotide auf mindestens einen ersten Träger aufgebracht werden, so dass das Array einzelne Spots mit einheitlichen Sequenzen für jedes selektierte Oligonukleotid umfasst,
- die Analyse der Bindungseigenschaften aller Sorten von auf dem Array der Amplifikate angeordneten Oligonukleotiden an die Aptamer-Zielstruktur, und die Zuordnung der analysierten Bindungseigenschaften zu den spezifischen Ortskennzeichnungen der Amplifikate und den Sequenzen der entsprechenden Sorten von Oligonukleotiden,- wobei zur Analyse der Bindungseigenschaften das Array der Amplifikate mit der Aptamer-Zielstruktur in Kontakt gebracht wird.
Dieses Verfahren zur Aptamer-Identifizierung/Gewinnung verläuft wesentlich schneller als der SELEX Prozess. Es ist nicht mehr erforderlich, mehrere Runden des Prozesses durchzuführen, sondern es genügt bereits eine einfache Selektion.
Die umfangreichen Arbeiten im Anschluss an den SELEX, die Klonierung zur Vereinzelung der bindenden Oligonukleotide aus dem angereicherten Pool entfallen. Nach der Klonierung erfolgt beim herkömmlichen Verfahren die Auswahl der weiter zu charakterisierenden Aptamere zufällig, so dass einzelne gute Binder "verloren" gehen können, weil sie zufälligerweise nicht ausgewählt, nicht weiter charakterisiert und daher nicht als Aptamere erfasst werden. Im hier beschriebenen neuen Prozess werden demgegenüber alle gebundenen Oligonukleotide hochparallel vereinzelt, sequenziert und analysiert. Die Aptamere werden als Binder mit den höchsten Affinitäten am Ende des Prozesses auf Basis der gemessenen Affinitäten ausgewählt. Somit erzielt die neue Methode aufgrund ihres Aufbaus eine erhöhte Ausbeute und erlaubt eine bessere Beurteilung der gewonnenen Aptamere.

Mit dem Verfahren ist es möglich, in kürzerer Zeit Aptamere zu identifizieren und gleichzeitig deren Sequenz und Bindungskonstante zum Target innerhalb des Prozesses zu bestimmen. Dabei bietet das Verfahren sämtliche Vorteile des SELEX-Verfahrens. Es besteht hier jedoch die Möglichkeit, schon nach einem ersten Selektionsschritt (Bindung, Entfernung der Nichtbinder, Elution der Binder vom Target), der gemäß den Schritten eines bekannten SELEX Verfahren durchgeführt werden kann, den Prozess zu beenden und bindende Aptamere zu identifizieren. Durch den optionalen Einsatz eines weiteren evaluierenden Bindungs- und Selektionsschritts noch während der Vorbereitung der Sequenzierung ergibt sich zudem die Möglichkeit einer deutlich höheren Spezifität als beim SELEX.

Weitere Vorteile, die mit der Erfindung und/oder mit speziellen nachfolgend beschriebenen Ausführungsformen erzielt werden können, sind:
- Die Möglichkeit der parallelen Sequenzierung und Untersuchung der Bindungseigenschaften einer sehr großen Anzahl von potentiellen Aptameren. Die Sequenzierung mittels nachfolgend beschriebener Varianten erlaubt die parallele Sequenzanalyse von bis zu 10⁶ Oligonukleotiden. In einem einzigen Durchgang können so bis zu 10⁶ Oligonukleotide selektiert, analysiert und validiert werden, so dass durch Affinitäts-Vergleich bei Inkubation mit dem Target am Ende eine Gruppe von Aptameren gefunden werden kann.
- Bei der räumlich getrennten Amplifikation wie hier verwendet, wird jedes Oligonukleotid einzeln amplifiziert. Bei der bisherigen Amplifikation konkurrieren die Oligonukleotide auch in der Amplifikation miteinander, so dass Oligonukleotide mit schlechter Amplifikationseffizienz, unabhängig von ihren Bindungsqualitäten, verloren gehen können.

Grundsätzlich gilt, dass die aufgezählten Verfahrensschritte nicht zwingend in der oben angegebenen Reihenfolge stattfinden müssen. Wenn sinnvoll und technisch möglich kann die Reihenfolge variiert werden. Insbesondere können die Schritte in der Reihenfolge variiert werden, die nach den Schritten a) dem in Kontakt bringen eines Gemisches von Oligonukleotiden mit einer vorbestimmten Aptamer-Zielstruktur, und b) dem Binden zumindest eines Teils der Oligonukleotide an die Zielstruktur, und dem Abtrennen der Oligonukleotide, die an die Aptamer-Zielstruktur gebunden haben stattfinden. Beispielsweise können das räumlich getrennte Amplifizieren, das Vergeben einer spezifischen Kennzeichnung, das Sequenzieren und die Analyse der Bindungseigenschaften der Aptamer-Zielstruktur in verschiedenen zeitlichen Reihenfolgen stattfinden, oder mehrere dieser Schritte können gleichzeitig stattfinden, wie aus den nachfolgenden Erläuterungen und Ausführungsbeispielen deutlich wird.

Die oben aufgezählten Schritte werden nun im Einzelnen erläutert.

Der Begriff "Aptamere" bezeichnet kurze einzelsträngige Nukleinsäure-Oligomere, die spezifisch an eine Zielstruktur oder ein Zielmolekül, auch bezeichnet als Target, binden können, beispielsweise an ein Protein, an niedermolekulare Verbindungen, wie organische Substanzen, Aminosäuren und Antibiotika, Nukleinsäuren, Viruspartikel oder (Mikro)Organismen, sowie weitere einleitend genannte Targets. Die Aptamer-Target-Bindung erfolgt wie einleitend genannt.

Als Ausgangssubstanz wird im Verfahren ein Gemisch von Oligonukleotiden eingesetzt. Der Begriff "Gemisch" bezeichnet eine Vielzahl Oligonukleotide mit verschiedenen Sequenzen.

Die Oligonukleotide weisen vorzugsweise einen oder mehrere variable Bereiche auf, vorzugsweise interne variable Bereiche. Zudem können Primerregionen vorhanden sein, vorzugsweise am 5' und 3' Ende der Oligonukleotide. Ein interner variabler Bereich, von denen einer oder mehrere vorhanden sein können, ist beispielsweise ein randomisierter Sequenz-Bereich, insbesondere ein Bereich, der eine kombinatorische Zufallssequenz ist oder aufweist. Ein interner variabler Bereich hat beispielsweise eine Länge von 10-80, vorzugsweise 20-80, noch mehr bevorzugt 40-80 Nukleotiden, wobei diese Längen lediglich beispielhaft angegeben sind und keine Beschränkung darstellen. Zwischen zwei variablen Bereichen kann ein Bereich mit einer festgelegten Sequenz angeordnet sein. Ein nicht beschränkendes Beispiel hierfür ist eine Fängersequenz, die nachfolgend noch erläutert wird.

Primerregionen dienen als Primerbindungsstellen für eine Amplifikation, vorzugsweise eine PCR-Amplifikation. Alternativ oder zusätzlich können die Primer zur Anbindung der Oligonukleotide an eine feste Phase, beispielsweise Platten, Beads, Chips oder sonstige Träger, dienen, wie nachfolgend noch beschrieben. Weiterhin lassen sich über die Primer vor, während oder nach dem Verfahren zusätzliche Modifikationen einbringen wie Fluoreszenz-Moleküle, Biotin-Label, Enzyme etc.

Die Ausgangssubstanz, d.h. das Ausgangsgemisch von Oligonukleotiden, wird auch als Ausgangsbibliothek, Oligonukleotidbibliothek, Bibliothek oder kombinatorische Zufallsbibliothek bezeichnet. Oligonukleotide mit der oben beschriebenen Struktur können von kommerziellen Anbietern bezogen werden. Die Variabilität einer Bibliothek liegt beispielsweise im Bereich von ca. 10¹⁵ verschiedenen Molekülen.

Bei den Oligonukleotiden kann es sich um einzelsträngige DNA (ssDNA), RNA, modifizierte ssDNA oder modifizierte RNA oder synthetische Nukleinsäure-analoge Moleküle (z.B. PNA) handeln. Im Laufe des Verfahrens liegen bei einzelnen Verfahrensschritten, insbesondere bei Amplifikationsschritten, zu den Oligonukleotiden komplementäre Gegenstränge vor. In den Amplifikaten sind nach Beendigung der Amplifikation die Oligonukleotide und ihre komplementären Gegenstränge enthalten, woraus die die gewünschte ssDNA für weitere Untersuchungen zur Target-Bindung gewonnen wird. Zur Untersuchung der Target Bindung werden die Oligonukleotid-Einzelstränge, insbesondere ssDNA, untersucht.

Das Verfahren ist insbesondere auch geeignet zur Anwendung für natürliche und artifizielle Nukleinsäuren, insbesondere für Spiegelmere, synthetische DNA-analoge Moleküle (PNA) etc. Das Prinzip der Spiegelmere wurde eingangs erläutert.

Das in Kontakt bringen der Oligonukleotide mit der Aptamer-Zielstruktur, das Binden zumindest eines Teils der Oligonukleotide an die Zielstruktur und das Abtrennen der Oligonukleotide, die an die Aptamer-Zielstruktur gebunden haben, von der Aptamer-Zielstruktur und von nicht an die Aptamer-Zielstruktur angebundenen Oligonukleotiden kann auf jede beliebige Art und Weise erfolgen, insbesondere so wie bereits aus den SELEX Prozessen aus dem Stand der Technik bekannt. Nachfolgend sind einige Varianten beispielhaft aufgezählt, ohne dass dies das Verfahren in irgendeiner Weise beschränkt.

Beispielsweise kann Target an eine feste Phase gebunden sein und mit gelösten Oligonukleotiden in Kontakt gebracht werden, wobei ein Teil der Oligonukleotide, die potentiellen Aptamere, an das Target binden. Die feste Phase kann Eigenschaften aufweisen, die eine leichte Abtrennung von der Flüssigphase mit nicht an Target gebundenen Oligonukleotiden erlauben. Beispielsweise kann die feste Phase magnetisch sein, beispielweise in Form magnetischer Partikel (magnetic beads). Mit einem Elutionsschritt, wie bereits aus SELEX Verfahren bekannt, können die Target-gebundenen Oligonukleotide von der Festphase und dem Target eluiert werden.

In einer anderen Variante ist an eine feste Phase, vorzugsweise in Form magnetischer Partikel, eine sog. Fängersequenz angebunden. Eine Bindung von einzelsträngigen Oligonukleotiden aus der Ausgangsbibliothek an die feste Phase erfolgt über die Fängersequenz (capture sequence). Die Fängersequenz ist zu einem Bereich innerhalb der einzelsträngigen Oligonukleotide der Ausgangsbibliothek komplementär. Mit Hilfe der Fängersequenz findet eine reversible Bindung zwischen Fänger und Oligonukleotiden statt. Das Target liegt frei in Lösung vor und steht für eine Bindung mit den Oligonukleotiden, den potentiellen Aptameren, zur Verfügung. Targetspezifische Oligonukleotide koppeln sich durch Ausbildung einer definierten zwei- oder dreidimensionalen Struktur von der Fängersequenz ab und sind zusammen mit dem Target in Lösung zu finden. Die feste Phase, an welche die nicht-targetspezifischen Oligonukleotide angebunden bleiben, kann zusammen mit diesen Oligonukleotiden abgetrennt werden.

Weitere Varianten sind Bindungs-Methoden die in den verschiedenen Modifikationen von SELEX-Prozessen verwendet werden, z.B. die Bindung zwischen ganzen Zellen und der Oligonukleotidbibliothek, dann Trennung der Nichtbinder mittels Zentrifugation und Elution der Binder mittels Hitze. Ein Überblick über SELEX-Verfahren ist enthalten in Stoltenburg, R.; Reinemann, C.; Strehlitz, B. (2007) SELEX - a (r)evolutionary method to generate high affinity nucleic acid ligands. Biomolecular Engineering 24, 381-403.

Wenn das Target an eine feste Phase gebunden ist, kann vor den oben beschriebenen Verfahrensschritten zusätzlich eine Negativ-Selektion zur festen Phase, d.h. eine Selektion gegen die feste Phase ohne angebundenes Target, erfolgen.

Es kann vor den oben beschriebenen Verfahrensschritten auch eine Selektion mit anderen unspezifischen Molekülen erfolgen. Ebenso können Counterselektionen mit verwandten, aber unerwünschten Targetmolekülen durchgeführt werden, um vor der Selektion aus der Oligonukleotid-Bibliothek entsprechende Binder abzureichern.

### Räumlich getrenntes Amplifizieren

Der Begriff "räumlich getrenntes Amplifizieren aller Oligonukleotiden" hat die folgende Bedeutung: Ein einzelnes Oligonukleotid ist ein Oligonukleotid mit einer bestimmten Sequenz. Alle Oligonukleotide werden auf eine Art und Weise so amplifiziert, dass jedes einzelne Oligonukleotid räumlich getrennt von den anderen amplifiziert wird und dass das erhaltene Amplifikat ebenfalls räumlich von allen anderen Amplifikaten getrennt bleibt. So findet keine Durchmischung der einzelnen Amplifikate statt.

Ein Amplifikat umfasst dabei eine Vielzahl Oligonukleotide, die durch die Amplifikation eines einzelnen Oligonukleotids mit einer bestimmten Sequenz erhalten werden. Jedes Amplifikat enthält vorwiegend, und je nach Amplifikationsmitteln und Bedingungen überwiegend, oder nahezu ausschließlich, oder ausschließlich, eine Sorte Oligonukleotide mit einer Sequenz, die mit der Sequenz des als Ausgangsmolekül (Matritze) verwendeten einzelnen Oligonukleotids übereinstimmt. Der Begriff "Sorte" bezieht sich somit auf die Sequenz eines Oligonukleotids. Eine Sorte ist durch eine bestimmte Sequenz gekennzeichnet. Das Amplifikat enthält nach dem Amplifikationsschritt zu den Oligonukleotiden komplementaräre Gegenstränge. Je nach äußeren Bedingungen wie z.B. Temperatur, pH, Salzgehalt etc. liegen Doppelstränge vor. Neben einer bestimmten Sorte Oligonukleotide mit identischer Sequenz können Oligonukleotide mit abweichender Sequenz, und entsprechende komplementäre Gegenstränge enthalten sein, die durch Kopierfehler bei der Amplifikation oder Zugabe und Einbau von artifiziellen Monomeren gebildet werden. Der Begriff Amplifikat wird aber in dieser Beschreibung auch für ein Produkt gebraucht, aus dem die Gegenstränge der Oligonukleotide entfernt wurden, z.B. zum Zweck der Sequenzierung oder für die Untersuchung zur Targetbindung der Oligonukleotide. In diesem Sinn kann der Begriff "Amplifikat" auch eine Vielzahl einzelsträngiger Oligonukleotide bedeuten, vorwiegend, nahezu ausschließlich oder ausschließlich, umfassend, oder bestehend aus, eine(r) Sorte Oligonukleotide mit identischer Sequenz.

Jedes bekannte Amplifikationsverfahren kann für das räumlich getrennte Amplifizieren angewandt werden, wobei die Polymerase-Kettenreaktion (PCR) bevorzugt ist.

Das räumlich getrennte Amplifizieren findet insbesondere in räumlich begrenzten Bereichen statt, die völlig abgeschlossen sein können oder nicht. Solche begrenzten Bereiche können insbesondere durch Phasengrenzen wie z.B. Festkörperstrukturen und Flüssigkeiten oder Flüssigkeit-Flüssigkeit-Phasengrenzen oder Flüssigkeit-Gas-Phasen erzeugt werden. Beispiele sind räumlich begrenzte Kavitäten räumliche Abgrenzungen, die die Diffusion in bestimmte Richtungen erleichtern und in andere Richtungen erschweren, wie beispielsweise eine Anordnung von Säulen oder Gräben, poröse Strukturen, oder molekulare Strukturen, die eine Diffusion in bestimmte Richtungen bevorzugen bzw. einschränken, wie z. B. Hydrogele, Aerogele oder Polymeroberflächen. Möglich sind auch geordnete oder ungeordnete Nano- oder molekulare Strukturen wie Polymerbranches, Dendrimere, Partikelarrays, Filtermembranen, Lipidmembranen (sphärisch oder planar), um räumlich begrenzte Bereiche zu implementieren. Ebenso können durch Kräfte und Felder oder eine Kombination aus Phasengrenzen und Feldern/Kräften entsprechende räumlich getrennte Bereiche geschaffen werden, wie z.B. elektrische Felder welche die Diffusion auf einen Bereich beschränken oder die DNA an die Oberfläche gebunden halten.

In bevorzugten Ausführungsformen ist das räumlich getrennte Amplifizieren ein Amplifizieren in einer Emulsion, ein digitales Amplifizieren, ein Amplifizieren in einer Kavität oder ein Amplifizieren an einer festen Phase, insbesondere eine Emulsions-PCR, eine Digital-PCR oder eine PCR an fester Phase.

Verfahren zum Amplifizieren an einer festen Phase (Solid-phase amplification) sind aus dem Stand der Technik bekannt. Hierbei werden zufällig verteilte Amplifikate auf einer Festphasenoberfläche, beispielsweise einer planaren Fläche, erhalten. Oligonukleotide können über geeignete Bindungssequenzen durch Basenpaarung an komplementäre Sequenzen gebunden werden, die an der Festphasenoberfläche angebunden sind. Die komplementären Sequenzen sind insbesondere Primersequenzen für eine PCR. Vorzugsweise sind Forward und Reverse-Primer zur Amplifikation der Oligonukleotide an der Festphasenoberfläche kovalent gebunden. Die benötigten Amplifikationsmittel für die PCR werden zugegeben. Das Verhältnis von Primern zu Template sowie die Dauer der PCR bestimmen die erhaltene Dichte von Amplifikaten auf der Festphasenoberfläche. Mit einem solchen Verfahren werden im Ergebnis räumlich getrennte Amplifikate auf der Festphasenoberfläche erhalten. Als Festphase werden häufig Glasplatten verwendet. Festphasenamplifikationen sind beschrieben in L. Metzker et al., Nature Reviews, Genetics, Vol. 11, 2010, 31-46, und der darin zitierten Literatur zur Festphasenamplifikationen, wie beispielsweise M. Fedurco et al., Nucleic Acids Res. 34, e22 (2006).

Eine bekannte Variante der Festphasenamplifikation ist die sogenannte "Bridge-amplifikation", die beispielsweise von der Firma Illumina in deren SOLEXA-Sequencer angeboten wird. An einer Festphasenoberfläche sind geeignete Forward und Reverse-Primer für die zu amplifizierenden Oligonukleotide kovalent angebunden. Nach erfolgter Bindung an die Oberfläche findet eine Amplifikation der Oligonukleotide statt. Der damit neu erzeugte komplementäre Strang ist kovalent an die Oberfläche gebunden und verfügt an seinem nicht gebundenen Ende über eine weitere Bindungsstelle. Diese kann nun mit dem passenden Primer auf der Oberfläche ebenfalls binden und eine weitere Amplifikation starten, die ihrerseits einen neuen Oligonukleotid-Strang erzeugt, der an einem Ende angebunden ist und am anderen freien Ende die ursprüngliche Bindungssequenz besitzt. So werden exponentiell immer mehr neue Stränge erzeugt, die an einem Ende fest gebunden sind und deren anderes Ende eine zwischenzeitliche Bindung an die Oberfläche erlaubt. Während der Amplifikation ist ein Strang am einen Ende fest (kovalent) und am anderen Ende lose (nicht-kovalent) gebunden und erzeugt so einen molekularen "Bogen", der als Bridge bezeichnet wird. Diesbezüglich beschreibt US 6,300,070 generell die Bridgeamplifikation und Abrams et al., Diagnostic and Gene Detection Ch. (1997) 171-189 beschreibt die Verwendung einer Bridgeamplifikation zur Sequenzierung. Dieses Verfahren kann dahingehend erweitert werden, als dass man den Gegenstrang nach der Amplifikationsreaktion entfernt und eine Sequenzierung vornimmt und nach der Sequenzierung eine Bindungsmessung gegen das Target vornimmt.

Werden für das Amplifizieren an einer festen Phase kommerziell erhältliche Systeme verwendet, an denen bestimmte Primersequenzen angebunden sind, so werden im erfindungsgemäßen Verfahren vorzugsweise Oligonukleotide eingesetzt, die kompatibel zur diesen Primersequenzen sind bzw. zu diesen Primern kompatible Bindungsstellen aufweisen. Ist dies nicht der Fall, so wird ein Ligationsschritt mit sogenannten Adaptern vorgenommen, die zu den Primern auf der festen Phase kompatibel sind. Wahlweise können die Oligonukleotide auch mittels einer Amplifikation endständig entsprechend verlängert werden. Dies könnte jedoch Einfluss auf die Bindecharakteristika besitzen. Daher ist es bevorzugt, dass die flankierenden Sequenzen der Oligonukleotide kompatibel zu den Primersequenzen der kommerziellen Sequenzierprozesse sind.

Das Amplifizieren in Emulsion ist vorzugsweise ein Emulsions-PCR. Als Basis dient vorzugsweise eine Wasser-in-ÖI-Emulsion. Tröpfchen aus wässriger Phase dienen als Mikroreaktor. Für diese Wasser-in-ÖI-Emulsion sind die Konzentrationen so gewählt, dass im Idealfall in jedem Tröpfchen aus wässriger Phase jeweils genau ein Oligonukleotid eingeschlossen wird. Weiterhin sind in der wässrigen Phase die erforderlichen Amplifikationsmittel, wie Primer und Polymerase, für die PCR enthalten. Nach Durchführung einer PCR werden räumlich getrennte Amplifikate erhalten, da die Tröpfchen aus wässriger Phase in der Ölphase voneinander getrennt vorliegen. Jedes Tröpfchen aus wässriger Phase enthält vorwiegend eine Sorte Oligonukleotide und komplementäre Gegenstränge. Eine solche Variante der Emulsions-PCR ist beispielsweise beschrieben in M. Nakano et al., Journal of Biotechnology 102 (2003) 117-124 und Williams et al., Nature Methods, Vol. 3, No. 7 (2006), 545-550.

In einer Ausführungsform der Erfindung werden die Oligonukleotide vor, während oder nach dem räumlich getrennten Amplifizieren an Festphasenpartikel angebunden, wobei Festphasenpartikel erhalten werden, an die jeweils nur ein Amplifikat mit einer Sorte Oligonukleotide angebunden ist. Dabei können wahlweise das amplifizierte Olignukleotid oder der komplementäre Gegenstrang oder beide kovalent an einen Partikel angebunden sein.

Diese Ausführungsform kann beispielsweise mit einer Variante der Emulsions-PCR kombiniert werden, bei der neben einem zweiphasigen System, wie Wasser-in-ÖI zusätzlich Festphasenpartikel eingesetzt werden. In dieser Variante einer Emulsions-PCR werden die Oligonukleotide über geeignete Bindungssequenzen an komplementäre Sequenzen gebunden, die an der Oberfläche der Festphasenpartikel angebunden sind. Oligonukleotide werden in wässriger Phase zusammen mit PCR-Amplifikationsmitteln und FestphasenPartikeln, die auch als Beads bezeichnet werden, gemischt und in Öl emulgiert, so dass eine Wasser-und-Partikel-in-ÖI-Emulsion entsteht. Für diese Wasser-in-ÖI-Emulsion sind die Konzentrationen so gewählt, dass im Idealfall in jedem Wassertröpfchen jeweils genau ein DNA-Strang und genau ein Partikel eingeschlossen werden. An der Oberfläche des Partikels ist entweder ein Primer, oder ein Primer-Paar (Forward und Reverse) kovalent angebunden. Wenn nur ein Primer (Forward oder Reverse) angebunden ist, wird der andere Primer gelöst in wässriger Phase bereit gestellt. Wenn ein Primer-Paar vorhanden ist, dann verläuft die Amplifikation entsprechend der oben beschriebenen Bridge-Amplifikation. Im Falle der Bridge-Amplifikation auf dem Bead kann, falls gewünscht, auch nach der Reaktion wahlweise einer der Primer und mittels des Primers kovalent gebundene Stränge (Olibonukleotide, Gegenstränge) wieder abgelöst werden. Im Ergebnis kann in allen Fällen durch die Amplifikation das gesamte Partikel mit Kopien des ursprünglichen Oligonukleotids bedeckt werden. Nach der Amplifikation können wahlweise die Oligonukleotide, die Gegenstränge oder beide kovalent an die Oberfläche gebunden sein. Nicht-kovalent gebundene Oligonukleotide oder nicht kovalent gebundene Gegenstränge lassen sich durch geeignete Umgebungsbedingungen (Salzgehalt, Temperatur etc.) in flüssige Phase überführen, dabei verblieben die kovalent gebundenen Oligonukleotide oder die kovalent gebundenen Gegenstränge als Einzelstränge auf dem Partikel. In der bevorzugten Ausführungsform ist nach einer Amplifikation und einer Entfernung der Gegenstränge eine Vielzahl Oligonukleotide kovalent auf dem Partikel angebunden. Bei dieser speziellen PCR ist es möglich jeweils genau ein Oligonukleotid so zu amplifizieren, dass es millionenfach auf eine kleine Polymerkugel (Bead) "aufkopiert" wird. Am Ende der Emulsions-PCR liegen also Millionen Beads vor, von denen jedes Millionen identischer Kopien eines jeweils anderen Oligonukleotids trägt. Emulsionspolymerisationen und -Amplifikationen unter Einbeziehung von Festphasenpartikeln (Beads) sind beschrieben in L. Metzker et al., Nature Reviews, Genetics, Vol. 11, 2010, 31-46, und der darin zitierten Literatur, wie beispielsweise D. Dressman et al., Proc. Natl. Acad. Sci. USA (2003) 100, 8817-8822, sowie in F. Diehl et al., Nature Methods (2006), Vol.3 No.7, 551-559.

Werden die für Amplifizieren in Emulsion kommerziell erhältliche Systeme mit Partikeln verwendet, an denen bestimmte Primersequenzen angebunden sind, so werden im erfindungsgemäßen Verfahren vorzugsweise Oligonukleotide eingesetzt, die kompatibel zu diesen Primersequenzen sind bzw. zu diesen Primern kompatible Bindungsstellen aufweisen. Ist dies nicht der Fall, so wird ein Ligationsschritt mit sogenannten Adaptern vorgenommen, die zu den Primern des kommerziell erhältlichen Systems kompatibel sind. Oder die Oligonukleotide können durch die geeignete Wahl von Primern während einer PCR direkt kompatibel gemacht werden. Hierbei enthalten diese Primer komplementäre oder identische Sequenzen sowohl zum kommerziellen System als auch zur Oligonukleotid-Bibliothek.

Bei einer Digital-PCR (dPCR) werden Oligonukleotide auf eine große Anzahl räumlich getrennter Amplifikationsbereiche verteilt und die PCR in jedem Bereich getrennt durchgeführt. Im Idealfall enthält ein Bereich nur noch entweder ein einzelnes Oligonukleotid (entsprechend einer 1) oder kein Oligonukleotid (entsprechend einer 0). Nach Durchführung der PCR enthalten die Bereiche entsprechend ein Amplifikat eines bestimmten Oligonukleotids oder kein Amplifikat. Zur Isolierung einzelner Oligonukleotide in getrennten Bereichen können beispielsweise Pikowell-Platten, Microwell-Platten, Kapillaren, oder Arrays miniaturisierter Kavitäten oder Nukleinsäure-bindende Oberflächen eingesetzt werden. Auch die PCR in einer Emulsion, wie zuvor beschrieben, wird gelegentlich zur digitalen PCR gerechnet. Die Durchführung einer digitalen PCR ist beispielsweise beschrieben in US 6,143,496, H. Nagai et al., Anal. Chem. 73, 2001; pp. 1043-1047; F. Shen et al., Lab Chip, 2010, vol. 10 (20), pp.2666-2672; B. G. Zimmermann et al., Prenat. Diagn., 2008, vol. 28 (12), pp. 1087-1093; Y. Gong et al., Lab Chip, 2010, vol. 10 (18), pp. 2334-2337; S. Lindstrom et al., Lab Chip, 2009, vol. 9 (24), pp. 3465-3471; J. S. Marcus et al., Anal. Chem., 2006, vol. 78 (3), pp. 956-958. Verfahren und Mittel durch Durchführung von digitalen PCR sind kommerziell beispielsweise von der Firma Fluidigm erhältlich.

### Vergeben einer spezifischen Kennzeichnung

An jedes räumlich getrennte Amplifikat wird eine spezifische Ortskennzeichnung vergeben anhand der es eindeutig identifizierbar ist. Die Kennzeichnung kann prinzipiell auf jede erdenkliche Weise erfolgen und ist insbesondere eine optisch, spektroskopisch, radioaktiv, elektronisch (z.B. mittels eines Barcodes oder eines RFID-Chips), anhand der räumlichen Position oder der Reihenfolge der Amplifikate detektierbare Kennzeichnung. In einer Variante können die Amplifikate, mit einer spezifischen Kennzeichnung versehen werden, beispielsweise einer zusätzlichen DNA Sequenz, einem Massenspektrometer-Tag oder einer Isotopenmarkierung. Wenn die Amplifikate an Träger gebunden sind, beispielsweise ein Festphasenpartikel, wie oben beschrieben, dann ist vorzugsweise eine Kennzeichnung oder Markierung an dem Träger oder dessen Position vorhanden. Dadurch, dass der das Amplifikat tragende Träger gekennzeichnet bzw. markiert ist, ist auch das angebundene Amplifikat gekennzeichnet. Mögliche, aber nicht abschließende Kennzeichnungen sind Barcodes, Farbcodes, Größe, Form, räumliche Position oder Anordnung, Massenspektrometer-Tags, Isotopenmarkierung, DNA-Markierung.

In den Verfahren der Erfindung werden die Amplifikate an verschiedenen Orten angeordnet und der Ort trägt eine eindeutige Kennzeichnung, beispielsweise eine Nummerierung oder Ortskoordinaten. In einer Ausführungsform umfasst das Verfahren die Anordnung der Amplifikate in örtlich getrennten Bereichen an, auf oder in einem oder mehreren ersten Träger(n), wobei ein Array der Amplifikate erhalten wird, in dem jedem Amplifikat als spezifische Kennzeichnung eine spezifische Ortskennzeichnung zugewiesen wird. In dieser Ausführungsform erfolgt die Sequenzierung der Oligonukleotide in mehreren Amplifikaten des Arrays. Die Sequenz der in einem Amplifikat enthaltenen Sorte Oligonukleotide wird der spezifischen Ortskennzeichnung des Amplifikats in dem Array zugeordnet. Bei der Analyse der Bindungseigenschaften der Aptamer-Zielstruktur werden einem positiven oder negativen Bindungsereignis die Sequenz der in einem Amplifikat enthaltenen Sorte Oligonukleotide und die spezifische Ortskennzeichnung des Amplifikats zugeordnet. Beispielhafte Träger sind, ohne Beschränkung darauf, Platten, Chips, Pikowellplatten, Gele, Mikroarrays, sogenannte Polonien (Literatur: http://en.wikipedia.org/wiki/Polony). Bridgeamplifikation, räumliche Anordnung von wässrigen Tropfen in einem Öl z.B. einem Schlauch (wie eine Perlenreihe sitzt dort Tropfen hinter Tropfen) oder einer Pikowell-Platte, in einer Ansammlung von Mikro-/Pikokavitäten eines mikrofluidischen Chips oder einer Oberfläche (mit und ohne Struktur).

Die Anordnung der Amplifikate an verschiedenen Orten an, auf oder in einem Träger kann bereits mit der Methode der Amplifikation kombiniert sein. Im Fall einer Amplifikation an einer festen Phase kann bereits die feste Phase der Träger sein und die Amplifikate sind in einem regelmäßigen oder unregelmäßigen Muster an verschiedenen Orten auf dem Träger angeordnet. Im Fall einer Digitalen PCR können die Amplifikate beispielsweise bereits in den jeweiligen Kavitäten an den Innenwände oder in den Kavitäten enthaltenen Nukleinsäurebindenden Oberflächen angebunden werden, so dass die Anordnung der Kavitäten als räumliche Markierung eine Zuordnung zur Sequenz erlaubt.

Im Fall einer Emulsions-PCR können die Amplifikate z.B. in gelöster Form in Tröpfchen aus wässriger Phase vorhanden sein, oder angebunden auf Festphasenpartikeln, die sich in wässrigen Tröpfen befinden, wie zuvor beschrieben. Die Tröpfchen mit gelöstem Amplifikat können auf einen flachen Träger verteilt und immobilisiert werden. Die Tröpfchen können beispielsweise durch eine hydrophile Beschichtung an den jeweiligen Positionen an der Oberfläche des Trägers fixiert werden, so dass dieselben in einer gegebenen räumlichen Anordnung auf dem Träger angeordnet sind. Beispielsweise kann der Träger ein regelmäßiges Muster aus hydrophilen Punkten aufweisen. Die Tröpfchen können auf dem Träger durch eine Ölphase voneinander separiert sein.

Tröpfchen können auch in Kavitäten eines Trägers, insbesondere einer Mikro-, Nano- oder Pikowellplatte oder eines Sequenzierchips, eingebracht werden. Ein Emulsionssystem mit Anbindung der amplifizierten DNA auf einem Chip ist beschrieben in Q. Ge et al., Molecules 2008, 13, 3057-3068.

In einer besonders vorteilhaften Ausführungsform liegen die Amplifikate auf der Oberfläche von Festphasenpartikeln vor, beispielsweise nach einer oben beschriebenen Emulsions-PCR, und die Festphasenpartikel (Beads) mit den Amplifikaten werden in örtlich getrennten Bereichen an, auf oder in einem oder mehreren festen Träger(n) angeordnet, wobei ein Array der Amplifikate erhalten wird. Die Beads können auf/in einem Gel, auf einer Platte oder einem sonstigen flachen Träger angeordnet und vorzugsweise immobilisiert werden, beispielsweise durch chemische Vernetzung auf einer Oberfläche mit chemischen funktionellen Gruppen. In einer besonders bevorzugten Ausführungsform werden die Beads in Kavitäten einer Mikro- oder Pikowellplatte oder eines Sequenzierchips eingebracht, beispielsweise durch Zentrifugation. Die Dimensionen von Beads und Kavitäten sind vorzugsweise so aufeinander abgestimmt, dass genau ein Bead in eine Kavität passt.

### Sequenzieren

Wie erwähnt, enthält jedes Amplifikat vorwiegend, oder je nach Amplifikationsmitteln und Amplifikationsbedingungen überwiegend oder fast ausschließlich, eine Sorte Oligonukleotide mit identischer Sequenz, die mit der Sequenz des als Ausgangsmolekül (Matrize) verwendeten einzelnen Oligonukleotids übereinstimmt. Um die Sequenz der Oligonukleotide in den Amplifikaten zu entschlüsseln werden sogenannte Sequenzierverfahren eingesetzt. Aus dem Stand der Technik sind Sequenzier-Maschinen bekannt, die sich einer Vielzahl von Reaktionsschritten und Techniken bedienen, um gewonnene Oligo- oder Polynukleotide, insbesondere DNA, zuerst einzufangen, zu vervielfältigen und dann Baustein für Baustein auszulesen. Mittels der gewählten Reaktionschemie der Sequenziermethode ist es dann möglich für jede einzelne Kavität, die DNA-Sequenz des darin enthaltenen Oligonulkeotids zu berechnen.

Im erfindungsgemäßen Verfahren werden die Oligonukleotide in räumlich getrennt vorliegenden und spezifisch gekennzeichneten Amplifikaten sequenziert. Der Sequenz der Sorte Oligonukleotide in einem Amplifikat wird die spezifische Ortskennzeichnung des Amplifikats zugeordnet.

Vor der Sequenzierung werden vorzugsweise im Amplifikat enthaltene, zu dem Oligonukleotid komplementäre Gegenstränge entfernt. Man erhält ein Produkt, aus dem diese Gegenstränge entfernt sind und das zum Zwecke der vorliegenden Erfindung weiterhin als Amplifikat bezeichnet wird.

Das Entfernen von Gegensträngen kann auf verschiedene Vorgehensweisen, die dem Fachmann an sich bekannt sind, erfolgen. Im Fall einer Emulsions-PCR mit Beads oder einer Festphasen-PCR, wie zuvor beschrieben, tragen die Beads bzw. die Oberflächen beispielsweise nur einen Primer, und der andere wird in Lösung zugegeben. So kann wahlweise der Oligonukleotid-Strang, welcher zuvor durch eine positive Bindung gegen das Aptamer-Target gewonnen wurde, oder der Gegenstrang auf der Oberfläche direkt durch die PCR verankert werden. Weitere Möglichkeiten sind durch Bindungssysteme wie z.B. Streptavidin-beladene Beads und Primer mit Biotin möglich. Einzelstränge können dann generiert werden, indem man unter denaturierenden Bedingungen wäscht, so dass der unerwünschte Gegenstrang sich einfach löst. Der Oligonukleotid-Strang bleibt kovalent an das Bead oder die Oberfläche gebunden und kann nicht verloren gehen. Geeignete Verfahren sind das Waschen mit Hochsalzpuffer, SSC Puffer, verdünnter Natronlauge und/oder Erhitzen.

Im erfindungsgemäßen Verfahren werden vorzugsweise Sequenziertechniken eingesetzt, bei denen die Amplifikate in örtlich getrennten Bereichen an, auf oder in einem oder mehreren festen Träger(n), auch bezeichnet als "Template" angeordnet sind. Insofern wird auf die obige Offenbarung zu diesem Aspekt verwiesen. Jedes auf dem Träger angeordnete Amplifikat wird getrennt sequenziert.

Solche Sequenziertechniken werden hier und in der einschlägigen Literatur zusammenfassend auch als "Sequenziertechniken der nächsten Generation" oder als NGS (Next Generation Sequencing) bezeichnet. NGS-Sequenziertechniken können auf verschiedenen Plattformen basieren, von denen einige kommerziell erhältlich sind, beispielsweise von den Firmen Roche (Roche/454), und Illumina/Solexa, Solid und Ion Torrent. Einen Überblick über NGS-Techniken gibt L. Metzker et al., Nature Reviews, Genetics, Vol. 11, 2010, 31-46.

Die NGS-Technologie, wie sie im System GS FLX 454 von Roche angewendet wird, basiert u.a. auf Techniken, die beschrieben sind in Wheeler, D.A. et.al, Nature 452 (2008) 872, Shendure J.; Ji H., Nature Biotechnology 26 (2008) 1135-1145, US 7,244,559, US 7,323,305, US 7,335,762, US 7,638,276. Ion Torrents Technologie basiert auf demselben Amplifikations und Positionierungssystem der Amplifikate, nutzt jedoch einen Feld-Effekt-Transistor um die biochemischen Reaktionen auszulesen (J. M. Rothberg, Methods and apparatus for measuring analytes using large scale fet arrays, Pub.-Date: 21.Mai. 2009; Pub.-No.: US 2009/0127589 A1). Die Geometrie der Anordnung der Amplifikate beider Technologien beruht jedoch auf einem identischen Prinzip und erlaubt so eine hochparallele Sequenzierung von bis zu 10⁶ DNA-Sequenzen mit jeweils ca. 500 Basenpaaren Länge und es sind auch kürzere Sequenzlängen möglich. Bei dieser Technik werden Beads, an deren Oberfläche jeweils ein Amplifikat eines bestimmten Oligonukleotids angebunden ist, in einen Sequenzierchip eingebracht. Der Chip enthält Millionen kleiner Kavitäten, die jeweils so groß sind, dass genau ein Bead in jede Kavität passt. Danach kann der Chip mit kleineren Beads verfüllt werden, die sämtliche Enzyme für die Sequenzierreaktion enthalten. Dies ist jedoch nicht unbedingt notwendig, sondern ist eine bevorzugte Ausführungsform der Sequenziertechnik selbst. Bei der nun folgenden Sequenzierung wird beim System 454 FLX ein Lichtsignal, bei Ion Torrent eine elektrische Ladung mittels H+ Ionen erzeugt, wenn eine DNA-Base eingebaut wird. Das Signal wird parallel für jede Kavität und damit jedes Bead aufgezeichnet und kann dann zu einer DNA-Sequenz umgerechnet werden. Somit erlaubt ein einziger NGS-Chip die Erfassung von bis zu 1 Milliarde Basenpaaren und mehr. Das Prinzip der Sequenzierung und die bei der Sequenzierung ablaufenden Prozesse sind u.a. erläutert in M. Ronaghi, Genome Res. (2001) 11, 3-11 und M. Margulies et al., Nature (2005) vol. 437, 376-380.

Alternativ zu den Bead-basierten NGS-Systemen gibt es noch solche, die eine Bridge-Amplifikation verwenden (z.B. Solexa). Dabei werden die Oligonukleotide auf einer Oberfläche statistisch verteilt angebunden und so amplifiziert, dass um jede Bindungsstelle einige 10⁵ Kopien des originalen Oligonukleotids entstehen. Diese einzelnen AmplifikationsBereiche sind zumeist kreisförmig, um das originale Oligonukleotid angeordnet. In der Ausführungsform des Sequenzierens der Firma Illumina wird diese räumliche Anordnung mittels des Einbaus unterschiedlicher fluoreszenter Nukleotide analysiert (US Pat. App 12878687, NUCLEIC ACID SEQUENCING SYSTEM AND METHOD). Jedoch ist eine Analyse dieser räumlichen Anordnung ebenfalls mittels der Detekionsprinzipien von 454 FLX oder Ion Torrent möglich, um daraus die Sequenz abzuleiten. Verwiesen wird auf die Erläuterungen in US 6,300,070, sowie L Metzker et al., Nature Reviews, Genetics, Vol. 11, 2010, 31-46 und Abrams et al., Diagnostic and Gene Detection Ch. (1997) 171-189. Zusätzlich wird bei dieser Sequenzier-Auslegung oft mittels einer Ligation von unterschiedlichen fluoreszent gelabelten Oligonukleotiden die DNA-Sequenz ermittelt. Auch hier kann die Oligonukleotid-Bibliothek so gewählt werden, dass sie dieser Sequenzierung zugänglich ist und einer späteren Bindungsmessung der Oligonukleotide/Aptamere zur Verfügung steht.

### Analyse der Bindungseigenschaften

Schließlich findet die Analyse der Bindungseigenschaften von allen Sorten von auf dem Array der Amplifikate angeordneten Oligonukleotiden an die Aptamer-Zielstruktur statt. Dazu werden Oligonukleotide, welche die Sequenz der Sorte Oligonukleotide aufweisen, die in den Amplifikaten enthalten ist, mit der Aptamer-Zielstruktur in Kontakt gebracht. In einer Variante des Verfahrens können die räumlich getrennten Amplifikate mit der Aptamer-Zielstruktur in Kontakt gebracht werden. Es ist nicht notwendigerweise das Amplifikat selbst und darin enthaltene Oligonukleotide, die mit der Aptamer-Zielstruktur in Kontakt gebracht werden. Der Begriff "Analyse der Bindungseigenschaften aller Sorten Oligonukleotiden" umfasst auch die Untersuchung von Oligonukleotiden, welche die gleiche Sequenz aufweisen wie die Oligonukleotide einer bestimmten Sorte und somit der Sorte zuzurechnen sind, welche aber nicht mehr in den gekennzeichneten Amplifikaten enthalten sind. Beispielsweise können Kopien der Oligonukleotide erzeugt und analysiert werden, wie nachfolgend in speziellen Ausführungsformen angegeben. Der Begriff "Analyse der Bindungseigenschaften aller Sorten Oligonukleotiden" umfasst also insbesondere, dass Oligonukleotide in den Amplifikaten mit der Aptamer-Zielstruktur in Kontakt gebracht werden und/oder dass Kopien der Oligonukleotide mit der Aptamer-Zielstruktur in Kontakt gebracht werden.

Der Begriff "Zuordnung der analysierten Bindungseigenschaften zu den spezifischen Kennzeichnungen der Amplifikate" bedeutet die Herstellung eines Bezugs einer analysierten Sorte Oligonukleotide zu dem Amplifikat, in dem die Sorte zu finden ist, mit Hilfe seiner Kennzeichnung. Aus der Sequenzierung ist die Sequenz der Sorte Olignukleotide bekannt, so dass im Ergebnis eine Zuordnung einer Bindungseigenschaft zu einer Sorte Oligonukleotide, ihrer Sequenz und der Kennzeichnung des entsprechenden Amplifikats erfolgt.

Bindungseigenschaften können anhand einer gewählten festgelegten Messwertskala klassifiziert werden. Eine Klassifizierung kann qualitativ und/oder quantitativ erfolgen. Beispielsweise kann man eine Reihung untersuchter Oligonukleotide vornehmen, von höchstem Signal zu niedrigstem. Eine qualitative Einordnung kann beispielsweise von keine Affinität bis sehr hohe Affinität reichen. Es kann ferner aufgrund eines oder mehrerer festgelegter Grenzwerte in Qualitätsstufen differenziert werden. Unter anderem bei einer Fluoreszenz und RlfS-Messung, wie nachfolgend beschrieben, lassen sich Bindungseigenschaften als Intensitäten mit relativer Einheit darstellen.

Das Verfahren kann auch bezeichnet werden als Verfahren zur Untersuchung von Oligonukleotiden zwecks Charakterisierung, Identifizierung, Gewinnung und/oder Selektion von Aptameren oder potentiellen Aptameren.

Bei oder nach der Analyse der Bindungseigenschaften werden Oligonukleotide als Aptamere identifiziert. Die Identifikation kann anhand einer Klassifizierung erfolgen wie oben beispielhaft beschrieben. Identifizierte Oligonukleotide sind vorzugsweise solche, die eine Bindungseigenschaft für die Zielstruktur aufweisen oder solche, bei denen ein Bindungsereignis feststellbar ist.
Das Verfahren kann einen Selektionsschritt umfassen, wobei als Aptamere identifizierte Oligonukleotide aus allen analysierten Oligonukleotiden selektiert werden.

Als Aptamere identifizierte Oligonukleotide können optional isoliert oder neu synthetisiert werden.

In einer Variante kann die Analyse der Bindungseigenschaften insbesondere umfassen:
i) die Zuordnung eines positiven oder negativen Bindungsereignisses zu der spezifischen Kennzeichnung des Amplifikats und der Sequenz der in dem Amplifikat enthaltenen Sorte Oligonukleotide, und
ii) die Identifizierung der Oligonukleotide, bei denen ein positives Bindungsereignis festgestellt wird, als Aptamere für die Zielstruktur.

Ein positives Bindungsereignis kann beispielsweise definiert werden als eine bestimmte detektierbare Signaldifferenz zum Grundsignal bzw. einer Negativkontrolle, bei der keine Bindung erfolgt. Darüber hinaus sind Differenzierungen über die Signalhöhe möglich.

Vor der Analyse der Bindungseigenschaften werden vorzugsweise im Amplifikat enthaltene, zu dem Oligonukleotid komplementäre Gegenstränge entfernt. Dies kann geschehen, wie vorangehend unter dem Punkt "Sequenzieren" beschrieben. Wenn die Gegenstränge bereits vor der Sequenzierung entfernt wurden, wie oben beschrieben, erübrigt sich dieser Schritt.

Die Analyse der Bindungseigenschaften kann mit allen Methoden erfolgen, die bereits aus dem Stand der Technik für Aptamere bekannt sind, insbesondere mit Methoden der Affinitätsbestimmung von Aptamer zu Target.

Ein Bindungsereignis von Aptamer an Target kann mit verschiedenen gängigen Methoden detektiert werden, die aus dem Stand der Technik, insbesondere Affinitätsmessungen zwischen Liganden, bereits bekannt sind. Wird das Aptamer mit dem Target in Kontakt gebracht, so bildet sich ein Aptamer-Target-Komplex durch Bindung des Aptamers an das Target. Die Bindung bzw. das Bindungsereignis kann beispielsweise visuell, optisch, photonisch, elektronisch, akustisch, opto-akustisch, nach Masse, elektrochemisch, elektrooptisch, spektrometrisch, enzymatisch, oder anderweitig chemisch, biochemisch oder physikalisch nachgewiesen werden.

Der Komplex kann durch eine Markierung sichtbar gemacht werden, beispielsweise in einer Indikatorreaktion nach einer direkten oder indirekten Kopplung eines Komplexpartners mit einer Markierungssubstanz. Entweder kann das verwendete Aptamer oder das Target mit einer Markierung (Label) versehen sein. Bevorzugte Markierungen (Label) sind visuell, optisch, photonisch, elektronisch, akustisch, opto-akustisch, nach Masse, elektrochemisch, elektrooptisch, spektrometrisch, enzymatisch, oder anderweitig physikalisch, chemisch oder biochemisch detektierbar. In einer Ausführungsform des Verfahrens wird die Markierung durch Lumineszenz, UV/VIS-Spektroskopie, enzymatisch, elektrochemisch oder radioaktiv nachgewiesen.

Lumineszenz betrifft die Emission von Licht. Im erfindungsgemäßen Verfahren finden beispielsweise die Photolumineszenz, die Chemilumineszenz und die Biolumineszenz zum Nachweis der Markierung Anwendung. Bei der Photolumineszenz oder Fluoreszenz erfolgt die Anregung durch Absorption von Photonen. Beispielhafte Fluorophore sind, ohne Beschränkung, Bisbenzimidazol, Fluoreszein, Acridinorange, Cy5, Cy3 oder Propidiumiodid, die kovalent an Aptamere gekoppelt werden können, Tetramethyl-6-Carboxyrhodamin (TAMRA), Texas Red (TR), Rhodamin, Alexa Fluor Farbstoffe (u.a. Fluoreszenzfarbstoffe verschiedener Wellenlängen von verschiedenen Firmen). Die Auswertung geschieht visuell oder mit entsprechenden Messgeräten, z.B. im Multilabel Counter, im Fluoreszenzmikroskop, oder durch Durchflusszytometrie, z.B. im Cytofluorimeter. Chemilumineszenz beschreibt die Emission sichtbaren Lichts als Folge einer chemischen Reaktion. Biolumineszenz bezeichnet die Emission sichtbaren Lichts als Folge einer Enzymreaktion, beispielsweise einer durch das Enzym Luciferase katalysierten Redoxreaktion.

Andere Markierungsstoffe sind Katalysatoren, kolloidale metallische Teilchen, z.B. Gold-Nanopartikel, kolloidale nicht metallische Teilchen, Quantum Dots, organische Polymere, Latexteilchen, oder Liposome mit signalerzeugenden Stoffen. Kolloidale Teilchen können kolorimetrisch nachgewiesen werden.

Einsetzbar als Marker sind auch Enzyme, deren enzymatische Reaktion durch den Verbrauch oder die Entstehung detektierbarer Substrate oder Produkte gekennzeichnet ist, wobei ohne Beschränkung eine optische oder elektrochemische Detektion angewandt werden kann. Ein Nachweis kann z.B. mit Enzymen als Markierungssubstanzen geführt werden, die Substrate zu farbigen Produkten umsetzen, vorzugsweise Peroxidase, Green Fluorescent Protein (GFP), Luciferase, [beta]-Galactosidase oder Alkalische Phosphatase. Beispielsweise wird das farblose Substrat X-Gal durch die Aktivität der [beta]-Galactosidase zu einem blauen Produkt umgesetzt, dessen Farbgebung visuell erfasst wird.

Der Nachweis kann auch mittels radioaktiver Isotope erfolgen, mit denen das Aptamer markiert ist, vorzugsweise 3H, 14C, 32P, 33P, 35S oder 125I, besonders bevorzugt 32P, 33P oder 125I. Bei der Szintillationszählung wird die vom radioaktiv-markierten Aptamer-Target-Komplex abgegebene radioaktive Strahlung indirekt gemessen. Eine Szintillatorsubstanz wird durch die radioaktive Strahlung angeregt. Beim Übergang in den Grundzustand wird die Anregungsenergie wieder als Lichtblitze freigesetzt, welche durch einen Photoelektronenvervielfacher (Photomultiplier) verstärkt und gezählt werden.

Die Aptamere können auch mit Digoxigenin oder Biotin markiert sein, die beispielsweise durch Antikörper oder Streptavidin gebunden werden, die wiederum eine Markierung tragen können, wie z.B. ein Enzymkonjugat. Die vorherige kovalente Verknüpfung (Konjugation) eines Antikörpers mit einem Enzym kann auf verschiedene bekannte Arten erfolgen. Der Nachweis der Antikörper-Bindung kann auch radioaktiv in einem RIA (radioactive immunoassay) mit radioaktiven Isotopen, vorzugsweise mit 125I, oder durch Fluoreszenz in einem FIA (Fluoroimmunoassay) mit Fluorophoren, vorzugsweise mit Fluorescein oder FITC, erfolgen.

In einer besonders vorteilhaften Ausführungsform wird zur Detektion eines Bindungsereignisses eine labelfreie Detektionsmethode eingesetzt, die auf der sogenannten bildgebenden reflektometrischen Interferenz-Spektroskopie (nachfolgend iRlfS) beruht. Die Methode beruht darauf, dass aufgrund von Interferenz an dünnen Schichten die Anlagerung von Molekülen an eine Oberfläche durch eine Verschiebung des Interferenzmusters erfasst werden kann. Das Verfahren kann auch zur Messung einer Bindungskinetik verwendet werden. Grundlagen des Verfahrens sind beschrieben in C. Hanel et al., Analytical and Bioanalytical Chemistry, 372 (2002) 91-100, J. Piehler et al. Analytical Biochemistry, 249 (1997) 94-102 und in US2010297671. Mit der iRlfS-Methode kann ein Auslesen von Bindungsreaktionen auf Mikroarrays in Echtzeit und labelfrei durchgeführt werden. In der vorliegenden Erfindung kann eine hochparallele Analyse von Bindungsereignissen und die Bestimmung von Bindungskonstanten erfolgen. Mit iRlfS kann in Echtzeit festgestellt werden, ob und wie stark und mit welchen kinetischen Parametern eine Interaktion zwischen den Target-Molekülen und jedem einzelnen Amplifikat stattfindet. Die Bindungskinetik und die Affinitätskonstanten aller Aptamere auf dem Array können parallel bestimmt werden. Zudem können parallele Untersuchungen zur Target-Affinität unter verschiedenen Bedingungen (Temperatur, pH, Salzgehalt etc.) und zur Spezifität erfolgen, wobei bis zu 10⁶ Amplifikate auf einem Array gleichzeitig analysiert werden können.

Weitere spezielle Ausführungsformen und Varianten, sowie Ergänzungen des zuvor beschriebenen Verfahrens werden nachfolgend angegeben:
Das in Kontakt bringen von Oligonukleotiden, welche die Sequenz der Sorte Oligonukleotide aufweisen, die in den Amplifikaten enthalten ist, mit der Aptamer-Zielstruktur, kann auf verschiedene Art und Weise erfolgen, wie nachfolgend beschrieben.

In einer Ausführungsform des Verfahrens wird ein Array von Amplifikaten, wie zuvor beschrieben, mit der Aptamer-Zielstruktur in Kontakt gebracht. Beispielsweise können Amplifikate, die in getrennten Kavitäten eines DNA-Chips, einer Mikrotiterplatte oder einer Mikrowellplatte, angeordnet sind, mit einer Flüssigkeit inkubiert werden, welche die Aptamer-Zielstruktur enthält.

### Erzeugen von Kopien und Derivaten des Arrays

In einer weiteren Ausführungsform umfasst das Verfahren
- das Erzeugen einer Kopie oder eines Derivats des Arrays der Amplifikate an, auf oder in einem zweiten Träger, wobei jedem Amplifikat der Kopie oder des Derivats als spezifische Kennzeichnung eine spezifische Ortskennzeichnung zugewiesen wird,
- das in Kontakt bringen der Kopie oder des Derivats des Arrays mit der Aptamer-Zielstruktur.

In dieser Ausführungsform werden direkt aus einem Amplifikat-Array (nachfolgend auch "Array") auf, an oder in einem ersten Träger, beispielsweise einem Sequenzierchip, Oligonukleotide oder Amplifikate oder Derivate der Oligonukleotide oder der Amplifikate auf einen zweiten Träger kopiert, wobei die Array-Struktur und damit die spezifische Ortskennzeichnung der Amplifikate des Arrays erhalten bleiben. Die spezifische Ortskennzeichnung, die den Amplifikaten auf der Kopie oder dem Derivat zugewiesen wird, beruht somit auf der spezifischen Ortskennzeichnung der Amplifikate in dem Array, das als Vorlage dient. Jeder Ortsposition des Originals kann eindeutig eine Ortsinformation auf der Kopie zugeordnet werden und umgekehrt.

Die Ausführungsform ermöglicht insbesondere die Herstellung einer Kopie eines Mikroarrays aus einer Anordnung von Oligonukleotid-Sequenzen aus einer Sequenzierung (z. B. Partikelarray in einem Sequenzierer von ABI oder Roche 454, oder einer planaren Oberfläche, wie aus einem Solexa von Illumina).

Unter einer Kopie kann erfindungsgemäß eine 1:1-Kopie der originalen Oligonukleotide verstanden werden, während unter Derivat eine Veränderung der originalen Oligonukleotide verstanden werden kann, beispielsweise Abkömmlinge oder Subsets der originalen Oligonukleotide.

Der Kopierprozess kann prinzipiell mehrfach wiederholbar sein, so dass beispielsweise aus einem Sequenzierchip eine Vielzahl identischer Oligonukleotid-Mikroarray-Kopien hergestellt werden kann. Die Vorlage bleibt dabei erhalten. Durch diese Ausführungsform wird die Aptamer-Identifizierung und die Untersuchung an vielen parallelen Proben nochmals erleichtert, da an verschiedenen Kopien unterschiedlichste Untersuchungen durchgeführt werden können und bei Bedarf neue Kopien erzeugt werden können. Beispielsweise können Bindungskinetiken und die Affinitätskonstanten aller Aptamere auf Kopien des Arrays parallel bestimmt werden. Zudem können parallele Untersuchungen zur Target-Affinität unter verschiedenen Bedingungen (Temperatur, pH, Salzgehalt etc.) und zur Spezifität erfolgen.

Insbesondere wird eine Kopie mit der labelfreien iRlfS-Technik untersucht, die oben erläutert wurde, was unter anderem eine Affinitätsanalyse aller Oligonukleotide auf der erzeugten Array-Kopie ermöglicht.

Vor dem in Kontakt bringen der Kopie oder des Derivats des Arrays mit der Aptamer-Zielstruktur werden vorzugsweise zu den Oligonukleotiden komplementäre Gegenstränge, die in den Amplifikaten der Kopie oder des Derivats vorhanden sein können, entfernt. Dies kann mit bekannten Methoden erfolgen, von denen unter dem Punkt "Sequenzieren" einige aufgezählt sind.

Insbesondere weist in dieser Ausführungsform der zweite Träger einen Bindungsadapter für Oligonukleotide oder Bindungseigenschaften für Oligonukleotide auf, und die Kopie oder das Derivat werden erzeugt, indem Oligonukleotide aus den Amplifikaten mittels der Bindungsadapter oder der Bindungseigenschaften an den Träger gebunden werden. Bindungsadapter sind vorzugsweise Oligonukleotide, meist bevorzugt mit der Länge und Funktion eines Primers für eine PCR. Bindungsadapter weisen vorzugsweise Sequenzen auf, die komplementär zu einem Teil der Sequenz des Oligonukleotid-Gegenstranges bzw Sequenzen enthalten, die identisch zum Oligonukleotid sind. Bei einer Amplifikation, die an dem zweiten Träger durchgeführt wird, kann der Bindungsadapter zu einem Oligonukleotidstrang verlängert werden, der kovalent an der Oberfläche des zweiten Trägers gebunden ist. Der erzeugte Oligonukleotid kann dabei identisch oder komplementär zum ursprünglichen Oligonukleotid sein. Bindungsadapter können auch Sequenzen aufweisen, die komplementär zu einem Teil der Sequenz des Oligonukleotid-Stranges sind. Bei einer Amplifikation, die an dem zweiten Träger durchgeführt wird, kann der Bindungsadapter zu einem Oligonukleotid-Gegenstrang verlängert werden, der kovalent an der Oberfläche des zweiten Trägers gebunden ist.

Diese Ausführungsform ermöglicht es ferner, Amplifikat-Arrays zu kopieren, auch ohne Kenntnis von der biochemischen Information oder der Sequenz zu haben. Dies ermöglicht es, bereits vor, nach oder während eines Sequenzierprozesses eine Kopie der dortigen Anordnung von Oligonukleotiden herauszukopieren und so eine Array-Kopie zu schaffen, ohne eine Kenntnis von den Sequenzen zu besitzen. Einzig die Zuordnung der Ortsinformation auf der Kopie muss eine Zuordnung zu den Sequenzinformationen des Sequenzier-Systems erlauben.

Die Ausführungsform ermöglicht es somit, direkt vor, nach oder während der zuvor beschriebenen Sequenzierung eine Array-Kopie zu fertigen, auch ohne Kenntnis der Sequenzen der einzelnen Oligonukleotide. Es ist möglich, die in dieser Ausführungsform hergestellte Kopie zu sequenzieren, um die Identität der Aptamere in den kopierten Amplifikaten festzustellen. Im Prinzip können somit an der Array-Kopie die gleichen Schritte vorgenommen werden, wie an der Array-Vorlage. Die Sequenzierung kann daher vor, während oder nach der Anfertigung der Kopie mit dem Original oder der Kopie durchgeführt werden.

Gemäß dieser Ausführungsform kann eine Kopie oder ein Derivat vor, während oder nach der Sequenzierung oder der Amplifikation hergestellt werden oder eine der Kopie kann wiederum als Vorlage einer Sequenzierung dienen. Vorzugsweise erfolgt das Binden der Kopien oder Derivate an den zweiten Träger gleichzeitig oder nach der Amplifikation. Eine Herstellung der Kopie oder des Derivats vor der Amplifikation kann zum Beispiel unter Verwendung des Solexa-Systems der Firma Illumina erfolgen. Hierbei werden vor, während oder nach der Amplifikation weiter Amplifikate des originalen Oligonukleotids erzeugt und dann auf einen zweiten Träger übertragen. Es können, bezogen auf den originalen Oligonukleotid, sowohl komplementäre als auch zum identische Amplifikate übertragen werden. Dieser zweite Träger kann beispielsweise ein Sequenzierchip eines 454 von Roche sein. Es ist auch denkbar vor, während oder nach einer Sequenzierung mit dem System 454 von Roche eine Kopie zu machen und diese dann in einem SOLEXA-Sequenzierer einzuspeisen und dann nochmals zu sequenzieren.

Eine spezielle Variante dieser Ausführungsform umfasst die folgenden Schritte
- die Bereitstellung zumindest eines räumlich begrenzten Amplifikationsmittelbereichs für jedes Oligonukleotid, der von den Amplifikationsmittelbereichen der anderen Oligonukleotide getrennt ist, wobei eine mit dem Bindungsadapter oder Bindungseigenschaften versehene Oberfläche des zweiten Trägers an die Amplifikationsmittelbereiche angrenzt,
- das Amplifizieren der Oligonukleotide mittels Amplifikationsmitteln in den Amplifikationsmittelbereichen zum Erzeugen von Amplifikaten der Oligonukleotide,
- das Binden von Einzelsträngen der Amplifikate oder Derivaten der Amplifikate an den zweiten Träger mittels des Bindungsadapters oder der Bindungseigenschaften, so dass eine räumliche Anordnung der Einzelstränge auf dem zweiten Träger der räumlichen Anordnung der Amplifikate in dem Array entspricht, aus dem die Einzelstränge stammen; und
- das Entfernen des zweiten Trägers mit den angebundenen Einzelsträngen von dem Array.

Die räumlich begrenzten Amplifikationsmittelbereiche können zumindest teilweise durch Mikro- oder Nanostrukturen in dem ersten, das Array tragenden Träger oder in dem zweiten, die Kopie oder das Derivat tragenden Träger definiert sein:
Das Amplifizieren nach der obigen Ausführungsform kann identisch sein mit dem räumlich getrennten Amplifizieren wie zuvor anhand des allgemeinen Verfahrens beschrieben. In diesem Fall findet das räumlich getrennte Amplifizieren in den Amplifikationsmittelbereichen statt.

Das Amplifizieren nach der obigen Ausführungsform kann auch ein weiterer (z.B. zweiter) Amplifikationsschrittt sein, der nach dem räumlich getrennten Amplifizieren, das zuvor anhand des allgemeinen Verfahrens beschrieben wurde, durchgeführt wird. Es ist zum Beispiel möglich, dass Beads, die nach einer ersten, räumlich getrennten Amplifikation und einer Entfernung von Oligonukleotid-Gegensträngen eine Vielzahl kovalent gebundener Oligonukleotide tragen, in den Amplifikationsmittelbereichen mit einem Amplikationsmittel in Kontakt gebracht werden, das bei dem Amplifizieren zur Synthese eines Oligonukleotid-Gegenstranges führt. Das Amplifizieren gemäß der obigen Ausführungsform kann also die Synthese von Gegensträngen bedeuten. Der Begriff "Amplifikat der Oligonukleotide" umfasst in diesem Fall die Gegenstränge. Durch eine geänderte Wahl der Primer ist es auch möglich zum originalen Oligonukleotid identische Oligonukleotide als Amplifikat zu erzeugen.

Bei den Einzelsträngen der Amplifikate, die an den zweiten Träger gebunden werden, kann es sich im Oligonukleotid-Einzelstränge und/oder deren komplementäre Gegenstränge handeln, vorzugsweise um die Gegenstränge. Das Binden erfolgt vorzugsweise durch Hybridisierung von Oligonukleotid-Einzelsträngen und/oder deren komplementären Gegensträngen an Bindungsadapter mit einer Sequenz

Ein Bindungsadapter an der Oberfläche des zweiten Trägers weist vorzugsweise eine Sequenz auf, die komplementär zu einem Teil der Sequenz des Oligonukleotid-Gegenstranges ist. Gegenstränge binden durch Hybridisierung an Bindungsadapter. Vorzugsweise wird danach eine weitere Amplifikation an dem zweiten Träger durchgeführt, bei der der Bindungsadapter zu einem Oligonukleotidstrang verlängert wird. Im Ergebnis werden kovalent an die Oberfläche des zweiten Trägers gebundene Oligonukleotide erhalten. Dieser weitere Amplifikationsschritt kann vor oder nach Entfernen des zweiten Trägers erfolgen. Nicht mehr benötigte Gegenstränge können entfernt werden, wobei übliche Verfahren (Waschen mit Puffer, Erhitzen) bereits erwähnt wurden.

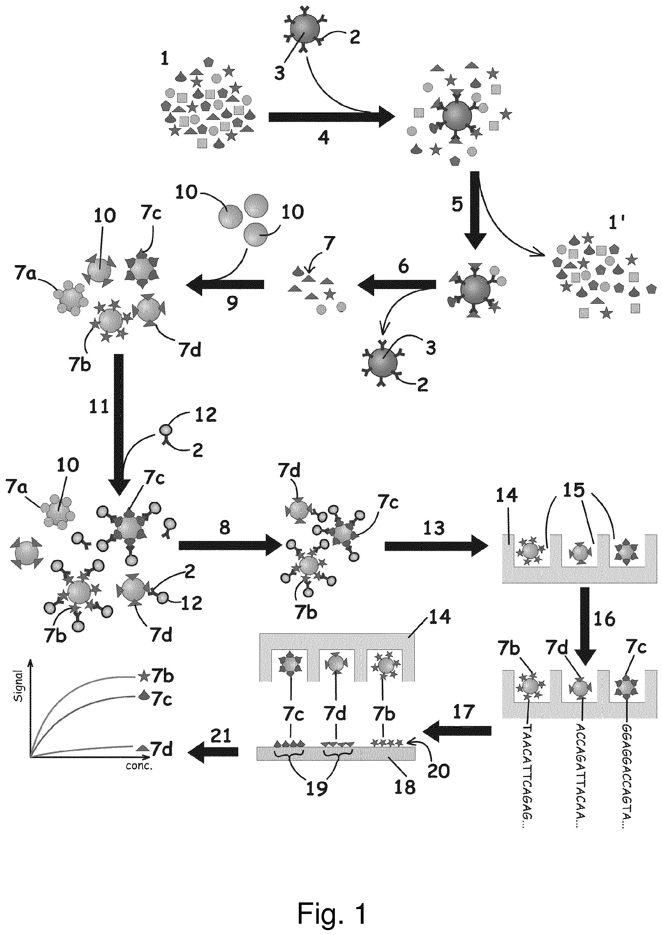

In einer Variante weist das Erzeugen zumindest eines räumlich begrenzten Amplifikationsmittelbereichs für jedes Oligonukleotid ein Bereitstellen der Oligonukleotide in jeweils zugeordneten separaten Ausnehmungen in dem ersten, das Array tragenden Träger, ein Einbringen des Amplifikationsmittels in die Ausnehmungen und ein Verschließen der Ausnehmungen durch den zweiten Träger auf. Beispielsweise kann der zweite Träger ein flacher Träger sein, der auf einen ersten Träger mit Kavitäten, wie eine Mikro- oder Pikowellplatte, aufgelegt wird, wobei sich in den Kavitäten die zu amplifizierenden Oligonukleotide befinden (z.B. bei digitaler PCR, wie oben beschrieben). Der zweite Träger verschließt die Kavitäten und es werden abgeschlossene Amplifikationsmittelbereiche gebildet. In den Kavitäten sind die erforderlichen Amplifikationsmittel vorhanden und bei der Amplifikation wird vorzugsweise zeitgleich eine Kopie der Amplifikate auf dem zweiten Träger gebildet. Der zweite Träger kann ein flacher Träger sein und darauf kann eine Kopie in Form eines planaren Arrays hergestellt werden. Ein solches Vorgehen ist beschrieben in WO2010100265. In einer Variante dazu sind Beads mit einem auf der Oberfläche gebundenen Oligonukleotid einer bestimmten Sequenz in Kavitäten eines ersten Trägers vorhanden und der zweite Träger wird als flacher Träger auf den ersten Träger mit Kavitäten aufgelegt. Anschließend wird eine Amplifikation durchgeführt und gleichzeitig eine Kopie auf dem zweiten Träger erzeugt. Ein Ausführungsbeispiel ist in WO2010100265 Bezug nehmend auf die dortigen Fig. 1a bis Fig. 1d beschrieben.

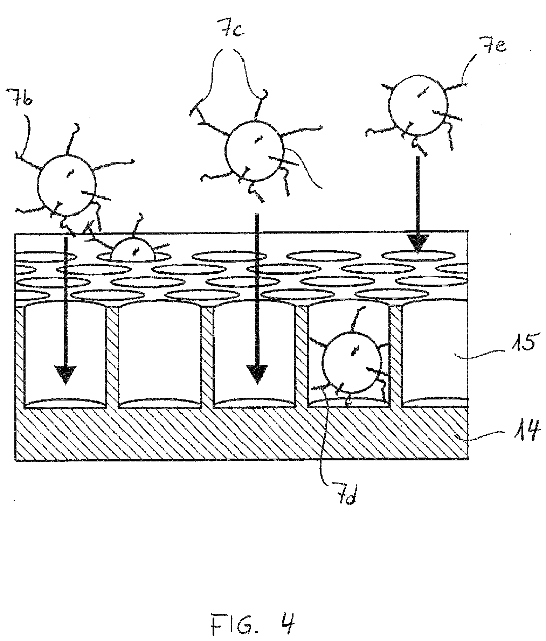

In einer weiteren Variante weist das Erzeugen zumindest eines räumlich begrenzten Amplifikationsmittelbereichs ein Bereitstellen des zweiten Trägers mit zumindest einer jedem Oligonukleotid zugeordneten Ausnehmung, in der der Bindungsadapter angeordnet ist, ein Einbringen des Amplifikationsmittels in die Ausnehmungen und ein Verschließen der Ausnehmungen mittels des ersten Trägers auf, so dass die Oligonukleotide dem Amplifikationsmittelbereich ausgesetzt sind. Ein Ausführungsbeispiel ist in WO2010100265 Bezug nehmend auf die dortigen Fig. 4a-4c beschrieben.

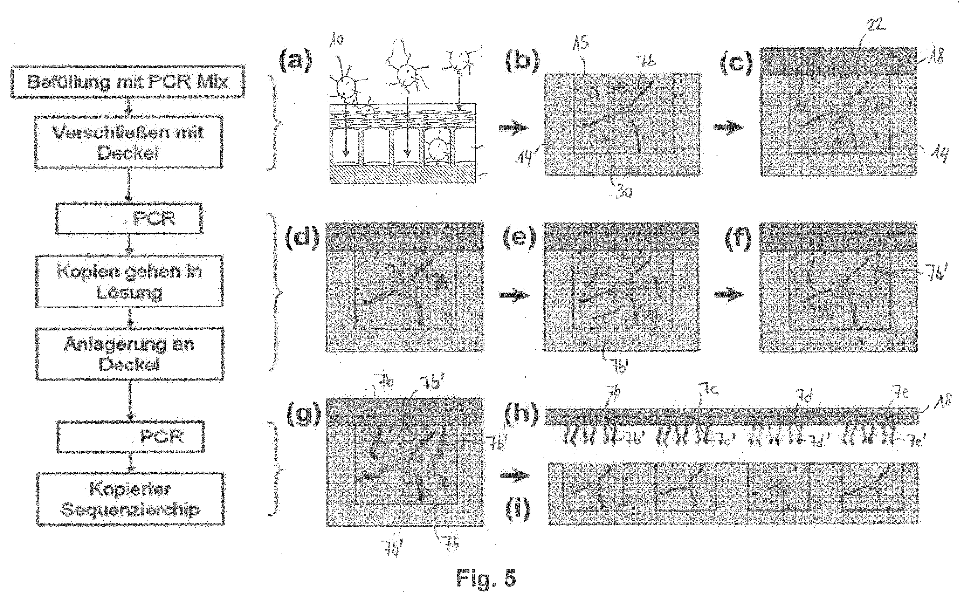

In einer weiteren Variante dieser Ausführungsform sind die räumlich begrenzten Amplifikationsmittelbereiche zumindest teilweise durch Phasengrenzen zwischen zwei Flüssigkeiten, einer Flüssigkeit und einem Gas oder einer physikalischen Grenze getrennt. Es ist auch denkbar, Emulsionströpfen auf einem ersten Träger anzuordnen, wobei jedes Tröpfchen ein Oligonukleotid einer bestimmten Sequenz und Amplifikationsmittel enthält, wie zuvor anhand der Emulsions-PCR beschrieben. Die Tröpfchen können durch eine hydrophile Beschichtung an den jeweiligen Positionen an der Oberfläche des ersten Trägers fixiert werden, so dass dieselben in einer gegebenen räumlichen Anordnung auf dem Träger angeordnet sind. Beispielsweise kann der Träger ein regelmäßiges Muster aus hydrophilen Punkten aufweisen. Der zweite Träger wird aufgedrückt, so dass er mit der Oberfläche, die vorzugsweise Bindungsadapter aufweist, in Berührung mit den Tröpfchen kommt. Bei einer Amplifikation in den Tröpfchen wird gleichzeitig eine Kopie auf dem zweiten Träger hergestellt. Ein Ausführungsbeispiel ist in WO2010100265 Bezug nehmend auf die dortigen Fig. 5a bis Fig. 5d beschrieben.

Während unterschiedlicher Kopierschritte können Abwandlungen der Arrays in chemischer Form hergestellt werden, die beispielsweise bestimmte Marker oder Sequenzen enthalten, vergleichbar einer Farbkopie, bei der nur der Gelbanteil kopiert wird.

Weitere Aspekte dieser Ausführungsform sind erläutert in WO002010100265.

### Weitere Verfahrensschritte mit einer Array-Kopie, Rückgewinnung und Selektion

In einer Ergänzung zur vorigen Ausführungsform umfasst das Verfahren die Rückgewinnung einer oder mehrerer Sorten Oligonukleotide aus einer Kopie, durch Entfernung der Oligonukleotide von dem ersten, dem zweiten Träger oder einer Kopie dieser Träger. Kopien der Träger weitere Kopien, die von einem Array an/auf/in einem ersten Träger gewonnen wurden, oder Kopien die von der Kopie auf dem zweiten Träger gewonnen wurden, wobei die bereits besprochenen Kopierverfahren einsetzbar sind. Eine bevorzugte Ausführungsform sieht jedoch die Verwendung von zweiten Trägern, insbesondere den DNA-Kopien einer Sequenzierung vor. In dieser Ausführungsform sind die Oligonukleotide oder deren Amplifikate so immobilisiert, dass sie ortsaufgelöst freigesetzt werden können. Dies kann bei einem kopierten DNA-Array auf einem zweiten Träger unter Verwendung von Photolinkern so umgesetzt werden, dass die Oligonukleotide dort zwar angebunden sind, sich aber durch Einstrahlen eines kurzwelligen Lasers ablösen lassen. Nach der Bindungsanalyse, beispielsweise mittels iRlfS, kann so gezielt ein Aptamer durch Einstrahlen des Lasers abgelöst und eluiert werden. Das Eluat steht dann direkt für weitere Schritte, wie eine PCR zur Verfügung. Somit kann direkt das Aptamer amplifiziert werden und muss nicht nochmals de-novo synthetisiert werden. Sofern ein Epitop-Binning vorgesehen ist, wie nachfolgend noch beschrieben, können rückgewonnene Aptamere als sekundäre Aptamere getestet werden und so können umgehend seine Bindungspartner für einen SandwichAufbau identifiziert werden.
Werden mehrere Aptamere jeweils zeitgleich freigesetzt, erlaubt dieser Aptamer-Pool zudem die Anwendung eines weiteren Selektionsschrittes (Bindung des Aptamer Pools mit dem Target, Entfernung der Nichtbinder, Elution der Binder vom Target), insbesondere in Verbindung mit einer Mutation des Pools. Jedoch kann man hierbei gezielt jede Sequenz dem Pool zuführen oder enthalten. Somit besteht eine wesentlich größere Einflussnahme als beim bisherigen SELEX-Verfahren auf den Aptamer-Pool für den nächsten SelektionsSchritt.

In noch einer Ergänzung zur vorigen Ausführungsform umfasst das Verfahren die Herstellung weiterer Oligonukleotide durch Amplifizieren von Oligonukleotiden der Kopie, die auf dem zweiten Träger gebunden sind. Die kopierten Arrays tragen sämtliche Aptamere an der Oberfläche. Es ist aufgrund des Herstellungsprozesses der Kopie nun möglich eine PCR auf der Oberfläche des Arrays durchzuführen und so wiederum einen Aptamer-Pool für eine nächste Runde zu gewinnen und diesen danach ebenfalls zu mutieren. Durch Auswahl passender Primer für PCR ist es möglich zudem Sub-Pools an Oligonukleotiden zu gewinnen.

In noch einer Ergänzung zur vorigen Ausführungsform ist es möglich, lösliche komplementäre DNA in Bezug auf die Aptamere, die während des Kopierprozesses entsteht, zurückzugewinnen und zu amplifizieren. Hierdurch erzeugt man wiederum einen Pool aller Aptamere, die in die Sequenzierung eingeführt wurden. Durch geeignete Wahl der Primer ist es möglich gezielt die Aptamere als ssDNA wieder zu gewinnen. Dieser Pool steht dann für eine weitere SELEX-Runde zur Verfügung und kann auch mutiert werden, um so nochmals eine neue Bibliothek zu erzeugen.

In noch einer Ergänzung zur vorigen Ausführungsform werden Sub-Pools der Aptamere aus der Kopie oder dem Überstand des Kopiervorgangs gewonnen. Da aufgrund der Sequenzierung die Sequenzen bekannt sind ist es möglich einzelne Sequenzmotive gezielt mittels geeigneter Primer zu amplifizieren. Dies ist sowohl aus dem Überstand des Kopiervorganges als auch dem erzeugten DNA-Array möglich. Somit können einzelne Sub-Pools des ursprünglich sequenzierten Aptamer-Pools erzeugt werden.

### Zusätzlicher Selektionsschritt

In einer weiteren Ausführungsform des Verfahrens, sind die Amplifikate an Festphasenpartikel angebunden, und das Verfahren umfasst weiterhin die folgenden Schritte:
- das in Kontakt bringen der Festphasenpartikel, an die jeweils ein Amplifikat mit einer Sorte Oligonukleotide angebunden ist, mit Aptamer-Zielstruktur,
- die Feststellung eines positiven oder negativen Bindungsereignisses der Aptamer-Zielstruktur an Oligonukleotide des Amplifikats, das an ein Festphasenpartikel angebunden ist,
- die Selektion von Festphasenpartikeln, bei denen ein positives Bindungsereignis feststellbar ist,

Die selektierten Festphasenpartikel, bei denen ein positives Bindungsereignis feststellbar ist, können in örtlich getrennten Bereichen an, auf oder in einem oder mehreren festen Träger(n) angeordnet werden, wobei ein Array der Amplifikate erhalten wird. Der Begriff "Selektion" bedeutet hier insbesondere die Abtrennung der Festphasenpartikel, bei denen ein positives Bindungsereignis feststellbar ist, von anderen Festphasenpartikeln.

Die Festphasenpartikel können aber auch vor Durchführung der obigen Verfahrensschritte bereits an, auf oder in einem festen Träger angeordnet sein und ein Array bilden. Der Begriff "Selektion" bedeutet dann insbesondere, dass weitere Verfahrensschritte, wie die nachfolgend beschriebene Sequenzierung, nur mit Festphasenpartikeln durchgeführt werden, bei denen ein positives Bindungsereignis feststellbar ist.

Wie zuvor anhand des grundsätzlichen Verfahrens beschrieben, wird ein Gemisch von Oligonukleotiden mit einer Aptamer-Zielstruktur in Kontakt gebracht und zumindest ein Teil der Oligonukleotide an die Zielstruktur gebunden. Anschließend werden die Oligonukleotide, die an die Aptamer-Zielstruktur gebunden haben, von der Aptamer-Zielstruktur und von nicht an die Aptamer-Zielstruktur angebundenen Oligonukleotiden abgetrennt. Hierbei handelt es sich um eine Selektion analog zu einem SELEX-Prozess, wobei das Target vorzugsweise immobilisiert ist und die Oligonukleotide in Lösung vorliegen. Aufgrund dieser Selektion ist davon auszugehen, dass die DNA jedes der Festphasenpartikel (Beads), an denen die erzeugten Amplifikate der selektierten Oligonukleotide angebunden sind, eine Affinität zum Target aufweist. Wenn jedoch die Oligonukleotide in der SELEX-analogen Selektion in Lösung gegen das immobilisierte Target getestet wurden, kann die Anbindung der Aptamere an die Beads die Affinität verändert haben. Um dies festzustellen, ist der in dieser Ausführungsform enthaltene weitere Selektionsschritt (auch bezeichnet als "Proofselektion") von Vorteil, da die Beads mit dem Target inkubiert werden, diejenigen Beads selektiert werden, bei denen ein positives Bindungsereignis feststellbar ist und im weiteren Verfahren, zur Sequenzierung und nachfolgenden Schritten, nur die Beads berücksichtigt werden, die auch in diesem weiteren Selektionsschritt gegen das Target binden.

Mit dieser Ausführungsform wird die Qualität der Binder verbessert und Nicht-Binder nochmals aussortiert. Mit diesem optionalen Schritt kann die Affinität der bereits selektierten Aptamere nochmals selektiert und damit in eine gewünschte Richtung verbessert bzw. optimiert werden. Durch die mögliche doppelte Selektion, zunächst freies Aptamer gegen gebundenes Target und dann gebundenes Aptamer gegen freies Target, kann eine höhere Trefferrate für Aptamere erreicht werden, die sowohl in immobilisierter Form als auch in ungebundener Form in Lösung ihr Target binden. Zudem ist besser gewährleistet, dass das Aptamer sowohl das frei in Lösung vorliegende als auch das immobilisierte Target bindet.

Es sind unterschiedliche Ausführungsformen der Proofselektion denkbar, von denen einige hier aufgezählt sind:
∘ Mittels fluoreszent gelabeltem Target kann eine einfache Affinitätsabschätzung über die Fluoreszenz des Beads erfolgen. Dies kann beispielsweise mit einem FACS-Gerät (z.B. Fluorescence assisted cell sorting) geschehen. Man kann dann die Beads in Pools mit unterschiedlicher Fluoreszenz einteilen und diese Pools dann getrennt sequenzieren um so verschieden gut bindende Aptamere zu erhalten.
∘ Bei kleinen magnetischen Beads auf die das Target immobilisiert wird, kann eine Bindung von Oligonukleotiden an das Target erfolgen. Danach kann eine magnetische Separation erfolgen. Nur Amplifikat-Beads, die das immobilisierte Target binden, interagieren mit den magnetischen Beads und werden dann separiert. Über eine Separation bei unterschiedlichen magnetischen Feldstärken kann ebenfalls eine Poolbildung in unterschiedlich affine Bereiche erfolgen.
∘ Weiterhin sind kompetitive Ansätze denkbar, bei denen beispielsweise ein Target und ein ähnliches Molekül verwendet werden und man dann auf gemeinsame oder unterschiedliche Affinität selektiert. Z.B. wenn das Target grün fluoreszent gelabelt und das ähnliche Molekül rot fluoreszent gelabelt ist, kann in nur Target bindende (grün), nur ähnliches Molekül bindende (rot) und beide bindende (gelb) Beads und damit Aptamere unterschieden werden.
∘ Auch eine Selektion nach On- oder Off-Kinetik ist denkbar. Hierzu werden die Beads entsprechend kurz inkubiert und dann umgehend vermessen (schnelle On-Kinetik ausschlaggebend für hohe Intensität der Fluoreszenz), oder lang und hochkonzentriert inkubiert, sehr lange und intensiv gewaschen und dann vermessen (langsame Off-Kinetik ausschlaggebend für hohe Intensität der Fluoreszenz).

### Identifikation von Aptamer-Bindunaspaaren

Das erfindungsgemäße Verfahren kann um weitere Schritte ergänzt werden, die der Identifikation von Aptamer-Bindungspaaren dienen. So betrifft die Erfindung auch ein Verfahren zur Identifikation von Aptamer-Bindungspaaren, die an verschiedene Stellen einer Aptamer-Zielstruktur binden, umfassend die Schritte des zuvor beschriebenen Verfahrens, und weiterhin umfassend:
a) das in Kontakt bringen des Arrays der Amplifikate oder der Kopie des Arrays der Amplifikate, mit der Aptamer-Zielstruktur und Bindung der Aptamer-Zielstruktur an Oligonukleotide, die in Amplifikaten des Arrays/der Kopie des Arrays enthalten sind,
b) das in Kontakt bringen des in a) erhaltenen Arrays oder der Kopie des Arrays mit einem Gemisch von Oligonukleotiden, und die Bindung zumindest eines Teils der Oligonukleotide an die Aptamer-Zielstruktur, die bereits auf dem Array gebunden ist,
c) die Elution der Oligonukleotide, die in b) nicht an die Aptamer-Zielstruktur gebunden haben,
d) das Entfernung der in b) gebundenen Oligonukleotide von der Aptamer-Zielstruktur
e) die Sequenzierung der in d) erhaltenen Oligonukleotide, und die Herstellung oder Isolierung der Oligonukleotide in sortenreiner Form,
f) das in Kontakt bringen eines Arrays der Amplifikate oder der Kopie des Arrays der Amplifikate mit der Aptamer-Zielstruktur und Bindung der Aptamer-Zielstruktur an Oligonukleotide, die in Amplifikaten des Arrays/der Kopie des Arrays enthalten sind,
g) das in Kontakt bringen des in f) erhaltenen Arrays oder der Kopie des Arrays mit einem sortenreinen Oligonukleotid aus e) und die Bindung des Oligonukleotids an eine oder mehrere Aptamer-Zielstrukturen, die bereits auf dem Array gebunden ist/sind,
h) die Analyse, an welche Aptamer-Zielstruktur(en) auf dem Array das sortenreine Oligonukleotid gebunden hat und die Zuordnung der Zielstruktur zu dem Oligonukleotid des Arrays, an das sie gebunden hat und dessen spezifischer Ortskennzeichnung in dem Array,
i) optional die ein- oder mehrfache Wiederholung der Schritte g) und h).

Dieses Verfahren wird auch als "Epitop-Binning" bezeichnet. In diesem Verfahren wird ein Sandwich-Assay mit Aptameren durchgeführt. Es werden zwei Aptamere identifiziert, die an unterschiedliche funktionelle Gruppen ("Epitope") des Targets binden.

Durch die Darstellung aller potentiell bindenden Aptamere auf einer Oberfläche ist es möglich, gezielt Aptamer-Paare aufzufinden, die das Target an jeweils unterschiedlichen funktionalen Gruppen binden. Diese Aptamere können dann in einem Sandwich-Assay Anwendung finden. Bisherige Methoden erlauben keine solch hochparallele Analyse von Bindungspaaren.

Das in Schritt b) eingesetzte Gemisch von Oligonukleotiden kann durch eine Rückgewinnung aus einer Aptamer-Kopie erhalten werden, wie weiter oben beschrieben.

Vorteilhaft aber nicht zwingend notwendig ist eine Überwachung der Bindung mittels der zuvor beschriebenen iRlfS-Technik. Hierzu wird das Array (mit dem immobilisierten Aptamer) oder die Kopie davon zunächst mit dem Target inkubiert, so dass sich ein Aptamer-Target-Komplex bildet. Mittels des iRlfS können alle Binder identifiziert werden. Nun wird dieses Array mit einem Aptamer-Pool, vorzugsweise aus einer Rückgewinnung, überschichtet. An den Stellen des Aptamer-Target-Komplexes an denen noch eine freie funktionelle Gruppe vorhanden ist, können passende Aptamere des Pools als sekundäre Aptamere binden. Mittels des iRlfS kann die Bindung sekundärer Aptamerer bestimmt werden. Danach wird die Oberfläche gewaschen um die Nicht-Binder zu entfernen. In einem Elutionsschritt werden nun die sekundären Aptamere gelöst und nochmals einer Sequenzierung unterzogen. Somit besitzt man alle Sequenzen der primären Aptamere und die Sequenzen der sekundären Aptamere. Die jeweiligen Sequenzen der sekundären Aptamere werden einzeln hergestellt. Nun wird nochmals ein kopiertes Array mit dem Target inkubiert und mit den sekundären Aptameren inkubiert. So ist es möglich zu jedem sekundären Aptamer das entsprechend passende primäre Aptamer zu finden und daraus ein Bindungspaar für das Target abzuleiten. Mittels des iRlfS lassen sich wiederum Kinetik oder Verhalten unter verschiedenen Bedingungen untersuchen und sich damit wiederum Aptamer-Paare mit gewünschten Eigenschaften auswählen.

### Arrays und deren Verwendungen, Vorrichtung

In einem weiteren Aspekt betrifft die Offenbarung ein zuvor beschriebenes Array aus Amplifikaten, die jeweils eine Sorte Oligonukleotide enthalten, oder eine Kopie eines Arrays von Amplifikaten, die jeweils eine Sorte Oligonukleotide enthalten, erhältlich nach den vorangehend beschriebenen Verfahren, wobei die Oligonukleotide Aptamere sind. Insbesondere weisen die Oligonukleotide einen oder mehrere variable Bereiche und Primerregionen auf, wie bereits zuvor anhand des erfindungsgemäßen Verfahrens beschrieben.

Weitere Aspekte der Offenbarung betreffen die Verwendung eines solchen Arrays oder einer Kopie eines solchen Arrays zur Analyse der Bindungseigenschaften von Aptameren an eine Aptamer-Zielstruktur, zur Identifikation von Aptameren für eine vorgegebene Zielstruktur oder zur Identifikation von Aptamer-Bindungspaaren, die an verschiedene Stellen einer vorgegebenen Aptamer-Zielstruktur binden. Verfahren zur Identifikation von Aptameren und von Aptamer-Bindungspaaren wurden zuvor in dieser Beschreibung angegeben.

Schließlich betrifft die Offenbarung auch eine Vorrichtung zur Gewinnung von Aptameren, umfassend
- eine Einrichtung zum in Kontakt bringen eines Gemisches von Oligonukleotiden mit einer Aptamer-Zielstruktur, und zum Binden zumindest eines Teils der Oligonukleotide an die Zielstruktur,
- eine Einrichtung zum Abtrennen der Oligonukleotide, die an die Aptamer-Zielstruktur gebunden haben, von der Aptamer-Zielstruktur und von nicht an die Aptamer-Zielstruktur angebundenen Oligonukleotiden,
- eine Einrichtung zum räumlich getrennten Amplifizieren von einzelnen Oligonukleotiden, die an die Aptamer-Zielstruktur gebunden haben, und zum Erhalt mehrerer räumlich getrennter Amplifikate, wobei jedes Amplifikat vorwiegend eine Sorte Oligonukleotide enthält,
- eine Einrichtung zum Vergeben einer spezifischen Kennzeichnung an mehrere der räumlich getrennten Amplifikate, sodass jedes der gekennzeichneten Amplifikate anhand seiner spezifischen Kennzeichnung eindeutig identifizierbar ist,
- eine Einrichtung zum Sequenzieren von Oligonukleotiden in mehreren gekennzeichneten Amplifikaten und eine Einrichtung zur Zuordnung der für ein Amplifikat spezifischen Kennzeichnung zu der Sequenz der Sorte Oligonukleotide in dem Amplifikat,
- eine Einrichtung zur Analyse der Bindungseigenschaften von Sorten Oligonukleotiden an die Aptamer-Zielstruktur, und zur Zuordnung der analysierten Bindungseigenschaften zu den spezifischen Kennzeichnungen der Amplifikate und den Sequenzen der Sorten Oligonukleotide.

Die Vorrichtung kann ferner so ausgestaltet sein und solche Einrichtungen aufweisen, dass sie zur Durchführung aller zuvor beschriebenen Ausführungsformen und ergänzender Schritte des Verfahrens geeignet ist. Ausführungsbeispiele erfindungsgemäßer Verfahren wurden oben erläutert. Ausführungsbeispiele entsprechender Vorrichtungen bzw. Einrichtungen zum Implementieren der erfindungsgemäßen Verfahrensschritte ergeben sich aus der Beschreibung oder sind für Fachleute offensichtlich. So bedarf es keiner weiteren Erläuterung, dass eine Vorrichtung geeignete Handhabungseinrichtungen aufweisen kann, um die physikalischen Entitäten, z. B. die verschiedenen Arrays, Träger oder Substrate, nach Bedarf zu positionieren. Ferner bedarf es keiner weiteren Erläuterung, dass geeignete Fluidikeinrichtungen vorgesehen sein können, um die jeweiligen Flüssigkeiten bzw. Mittel an den erforderlichen Positionen bereitzustellen. Ferner ist es für Fachleute offensichtlich, dass eine entsprechende Steuerung vorgesehen sein kann, um die Vorrichtung zu steuern, um die erfindungsgemäßen Verfahren auszuführen. Einrichtungen zum Erzeugen einer zur Durchführung der Verfahren erforderlichen Umgebung, beispielsweise Temperaturgeber, können ebenfalls vorgesehen sein.

Die Erfindung wird nachfolgend anhand von Ausführungsbeispielen beschrieben.

### 1. Darstellung des Verfahrens

Nachfolgend ist ein Beispiel eines bevorzugten Verfahrens anhand der Fig. 1 erläutert.

### • Vorbereitung: Design der Oligonukleotidbibliothek:

Vorzugsweise wird eine Oligonukleotidbibliothek entworfen, die kompatibel mit einer Next Generation Sequenzierung (NGS) ist. Die Primer-Regionen der Bibliothek werden kompatibel zur Sequenzierung (NGS) gewählt, um Target-bindende ss-DNA Oligonukleotide direkt in den Sequenzierungsprozess mittels NGS einzugeben. Diese Oligonukleotid-Bibliothek kann für verschiedene Verfahren (z.B. für verschiedene Targets) verwendet werden.

### • Selektion der Aptamere für immobilisiertes Target

Eine Oligonukleotidbibliothek 1, bestehend aus ∼10¹⁵ unterschiedlichen ssDNA-Molekülen, wird mit einem Target 2, welches immobilisiert an einem Target-Bead 3 vorliegt, in einem Bindungsschritt 4 in Kontakt gebracht. Hierdurch können einzelne Oligonukleotide an Targetmoleküle und damit an die Target-Beads 3 anbinden. Durch einen Waschschritt 5 werden die Oligonukleotide 1' entfernt, die kaum oder überhaupt nicht an Targetmoleküle gebunden haben. Alternativ zu einer Oligonkleotid-Bibliothek aus ssDNA kann eine RNA-Oligonukleotidbibliothek verwendet werden.

### • Elution der gebundenen Oligonukleotide vom Target

Die verbleibenden, gebundenen Oligonukleotide werden durch einen geeigneten Elutionsschritt 6 aus ihrer Targetbindung und damit von den Target-Beads 3 gelöst.

### • Erzeugung von Oligonukleotid-Beads mittels Emulsions-PCR

Die eluierten Oligonukleotide 7 werden einer Emulsions-PCR 9 unterzogen. Zunächst wird jedes der Oligonukleotide auf genau ein Bead 10 kopiert und anschließend vervielfältigt. Ein Bead 10 trägt das Oligonukleotid 7a, ein weiteres Bead 10 das Oligonukleotid 7b, ein weiteres Bead 10 das Oligonukleotid 7c und noch ein weiteres Bead 10 das Oligonukleotid 7d. Die Beads tragen kovalent angebundene Adaptersequenzen (nicht gezeigt), die bei allen Beads identisch sind und an welche die Oligonukleotide mittels Basenpaarung anbinden. In der gewählten Darstellung tragen zwei Beads 10 das Oligonukleotid 7d, das mehrfach im Pool vorhanden war. Entscheidend ist, dass jedes Bead 10 nur ein bestimmtes Oligonukleotid trägt. Mittels einer Öl-Emulsion wird erreicht, dass je ein Oligonukleotid-Strang und ein Bead eingeschlossen werden. Nach der PCR trägt jedes Bead 10 Millionen Kopien genau eines Oligonukleotids, wobei in der hier gewählten schematischen Darstellung pro Bead 10 vier bis sechs Kopien eines Oligonukleotids gezeigt sind. Es entsteht ein Pool von Oligonukleotid-Beads 10. Die DNA kann so millionenfach auf dem Bead vervielfacht werden. Wenn eine RNA-Oligonukleotidbibliothek eingesetzt wurde, wird eine Reverse Transkriptase-Emulsions-PCR durchgeführt.
Je nach verwendetem Bead kann wahlweise ein Forward oder ein Reverse-Primer auf dem Bead sitzen. Im Falle eines 454 von Roche handelt es sich dabei um die Sequenzieradapter A bzw. B.

In der gezeigten Ausführungsform werden ein einen optionaler, weiterer Bindeschritt 11 und ein Selektionsschritt 8 (Proof Selection) durchgeführt. Im Bindeschritt 11 wird zu den Beads 10 mit der Oligonukleotiden 7 gelöstes Target 2 gegeben, das einen Fluoreszenzmarker 12 trägt. Das markierte Target bindet nur an einen Teil der Oligonukleotide, in der hier gewählten Prinzipdarstellung an Oligonukleotid 7b, 7c und 7d, aber nicht an 7a. In dem Selektionsschritt 8 werden die Beads 10 mit den Oligonukleotiden 7b, 7c und 7d und dem angebundenen Target 2 selektiert.

### • Seguenzierung der Oligonukleotid-Beads

Die selektierten Oligonukleotid-Beads mit den Oligonukleotiden 7b, 7c und 7d werden in einen Next Generation Sequenzierprozess eingespeist. Zur Sequenzierung werden die Beads im Befüllschritt 13 mit zusätzlichen kleineren Beads (nicht gezeigt) in die Kavitäten 15 einer Pikowellplatte 14 eingefüllt, hier bezeichnet als Chip. Die kleinen Beads tragen ein Enzymsystem, welches im nachfolgenden Sequenzierschritt 16 Licht beim Einbau von DNA-Bausteinen erzeugt. Das Lichtsignal ist dabei quantitativ zur Anzahl der eingebauten DNA-Bausteine. Der Chip 14 sieht vergrößert wie eine Bienenwabe aus. Jede Wabenzelle ist eine Kavität 15, die mit einer Lichtfaser (nicht gezeigt, Durchmesser -45 µm) gekoppelt ist die ein Bead mit Oligonukleotid enthält. Im Ergebnis erhält man die Sequenzen aller Oligonukleotide des im Selektionsschritt 8 selektierten Pools.

Bei der Hochdurchsatz-Sequenziertechnik handelt es sich um einer Next Generation Sequenziertechnik, hier einen 454 Sequenzierer (Roche). Alternativ können andere Sequenzierer, wie die von Illumina, Solexa, Solid, Ion Torrent, ABI etc. eingesetzt werden.

### • Kopieren der Oligonukleotide des Sequenzierchips zu einem Oligonukleotid-Array

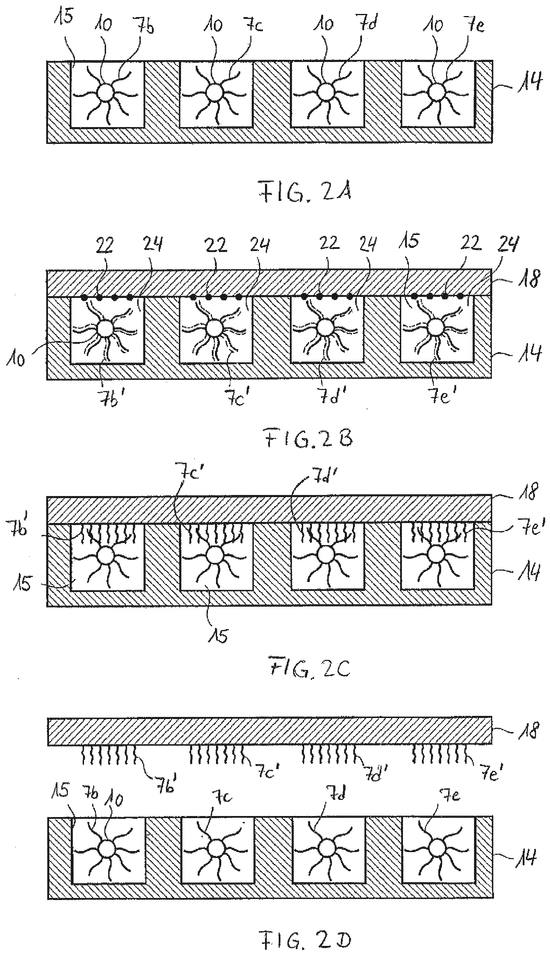

Mittels der DNA-zu-DNA-Kopiertechnik werden die Oligonukleotide des selektierten Pools aus dem Sequenzierchip in einem Kopierschritt 17 in Form eines Arrays 20 auf einen planaren Träger 18 kopiert. Das Array 20 auf dem Träger 18 enthält sämtliche sequenzierten Oligonukleotide, hier beispielhaft 7b, 7c, 7d. Damit können bis zu 10⁶ Spots (Bezugszeichen 19) mit zunächst doppelsträngiger DNA erzeugt werden. Durch entsprechende Aufarbeitungsschritte wird ss-DNA und damit für eine Targetbindung funktionelle DNA hergestellt, wobei jeder Spot 19 die einheitlichen Sequenzen eines selektierten Oligonukleotids trägt. Der Kopierschritt ist nachfolgend anhand der Fig. 2-5 näher erläutert.

### • Vermessung und Analyse der erhaltenen Arrays

Anschließend erfolgt in 21 eine Analyse der Bindung aller Oligonukleotide des Arrays 20 für das entsprechende Target. Für einen ersten Überblick kann die Messung beispielsweise mit einem fluoreszenten Target durchgeführt werden. Bindende Aptamere sind dann durch ihre erhöhte Fluoreszenz identifizierbar.

In der bevorzugten Ausführungsform erfolgt die Bindungsdetektion mittels der in der allgemeinen Beschreibung erläuterten iRlfS-Technik, deren Durchführung beschrieben ist in C. Hanel et al., Analytical and Bioanalytical Chemistry, 372 (2002) 91-100, J. Piehler et al. Analytical Biochemistry, 249 (1997) 94-102 und US2010297671. Hiermit kann labelfrei und in Echtzeit festgestellt werden, ob und wie stark und mit welchen kinetischen Parametern eine Interaktion zwischen den Target-Molekülen und jedem einzelnen DNA-Spot stattfindet. Die Bindungskinetik und die Affinitätskonstanten aller Aptamere auf dem Array werden parallel bestimmt. Zudem können parallele Untersuchungen zur Target-Affinität unter verschiedenen Bedingungen (Temperatur, pH, Salzgehalt etc.) und zur Spezifität erfolgen, wobei bis zu 10⁶ DNA-Spots auf dem Array gleichzeitig analysiert werden.

### • Zuordnung der Aptamersequenzen

Die Affinitätskonstanten der einzelnen Oligonukleotide werden vergleichend bewertet. Aptamere sind diejenigen Oligonukleotide mit den höchsten Affinitäten zum Target. Ihre Sequenzen können sofort aus den Ergebnissen des Sequenzierungsschrittes 16 entnommen werden, denn durch die DNA-zu-DNA-Kopiertechnik in Schritt 17 ist gewährleistet, dass sich die Aptamer-Spots 19 des Aptamer-Arrays 20 an den gleichen Positionen wie im Sequenzierchip 14 befinden. Somit ist eine eindeutige Zuordnung zwischen Bindung und Sequenz gegeben.

### 2. Kopierschritt

Der im obigen Beispiel verwendete Kopierschritt 17 ist anhand weiterer Ausführungsbeispiele a) und b) anhand der Fig. 2A-2D, 3, 4 und 5 genauer erläutert.

### Ausführungsbeispiel a) (Fig. 2A-2D, 3, 4)

Die Bezugszeichen für identische Gegenstände sind gleich gewählt wie in der Fig. 1.

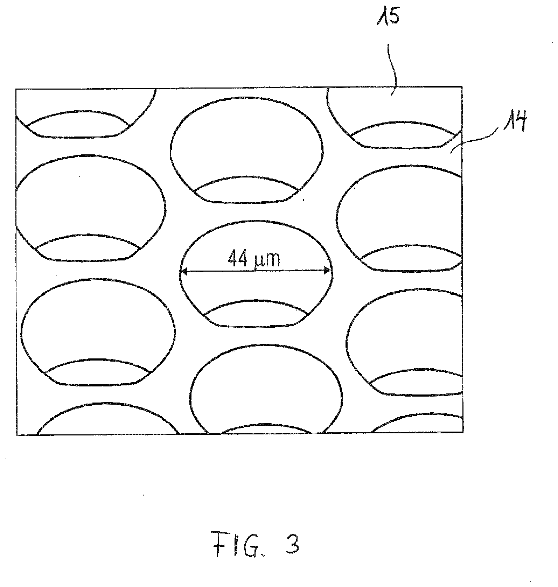

Der Sequenzierer-Chip 14 weist eine Mehrzahl von Mikrokavitäten 15 auf. Eine schematische Draufsicht auf einen Ausschnitt des Sequenzierer-Chips 14 (erster Träger) mit den Mikrokavitäten 15 ist in Fig. 3 gezeigt. Die Mikrokavitäten können beispielsweise eine Abmessung von 44 µm aufweisen, wie in Fig. 3 gezeigt ist. Der Sequenzierer-Chip kann beispielsweise ein Sequenzierer-Chip (GS Titanium 2005 und GS FLX Titanium 2008) des 454-Sequenzers der Firma Roche sein.

In jede der Kavitäten 15 ist ein Partikel 10 verbracht, wobei jeder dieser Partikel Millionen Kopien jeweils eines Oligonukleotids 7b, 7c, 7d (vgl. Fig. 1) du 7e (nicht gezeigt in Fig. 1) trägt. Die Vielzahl Kopien ist erhalten durch eine Emulsions-PCR, die eine räumlich getrennte Amplifikation ist und anhand des vorherigen Beispiels erläutert wurde. Eine schematische Querschnittdarstellung des Sequenzierer-Chips 14 mit den Kavitäten 15, in die die Partikel 10 mit den Oligonukleotiden 7b, 7c, 7d, 7e eingebracht werden, ist in Fig. 4 gezeigt.

Bei dem in den Fig. 2 bis Fig. 4 dargestellten Ausführungsbeispiel wird dieser Chip als primäres Array für die Herstellung einer Kopie verwendet. Es sollen die Oligonukleotide aus den Kavitäten 15 herauskopiert werden. Die Kavitäten werden zu diesem Zweck zunächst mit einem Amplifikationsmittel befüllt, beispielsweise einem PCR-Mix. Danach wird, wie in Fig. 2b gezeigt ist, ein Träger 18 (zweiter Träger) aufgebracht, der die Kavitäten 15 verschließt und zu dem Amplifikationsmittel passende Bindungsadapter trägt, die in 2b schematisch als Punkte 22 gezeigt sind. Nach dem Verschließen der Kavitäten 15 durch den Deckel 18 ist somit für jede Probe, d. h. jedes Partikel 10 mit daran gebundenen DNA-Strängen 7b, 7c, 7d, 7e ein räumlich begrenzter Amplifikationsbereich 24 erzeugt, der von den Amplifikationsbereichen 24 der anderen Proben getrennt ist. Die Bindungsadapter 22 grenzen an diese Amplifikationsbereiche 24 an. In diesem Beispiel sind die Bindungsadapter 22 Primer, welche mit den Gegensträngen zu den Oligonukleotiden 7b, 7c, 7d, 7e hybridisieren. Diese Primer 22 sind auch Bindungsstellen für die DNA-Polymerase. Fig. 2b zeigt bereits den Zustand nach dem Amplifikationsschritt (hier der zweite Amplifikationsschritt nach der bereits zuvor erfolgten Emulsions PCR, bei welcher Beads mit einer Vielzahl Oligonukleotide erhalten wurden, s.o.), in dem komplementäre Gegenstränge 7b', 7c', 7d', 7e' der Oligonkleotide 7b, 7c, 7d, 7e gefertigt werden. Diese Gegenstränge sind in Fig. 2b als gestrichelte Linien 7 dargestellt. Die Gegenstränge 7b', 7c', 7d', 7e' stellen eine Negativ-Kopie der Oligonukleotide dar.

Nachfolgend werden die erstellten Negativkopien 7b', 7c', 7d', 7e' der Oligonukleotide von den Partikeln 10 gelöst, was beispielsweise durch Erwärmen des Sequenzierer-Chips und somit der Kavitäten, in dem dieselben angeordnet sind, erfolgen kann. Anschließend hybridisieren die gelösten Kopien 7b', 7c', 7d', 7e' mit den Bindungsadaptern 22, was beispielsweise durch Abkühlen des Sequenzierer-Chips unterstützt werden kann. Das Ergebnis des Ablagerns der Kopien 7b', 7c', 7d', 7e' an den Bindungsadapter 22 und somit den Träger 18 ist in Fig. 2c dargestellt. Bei diesem Schritt der Übertragung der Negativ-Kopien an den Träger 18 bleibt die Ortsinformation bzw. die Registrierung erhalten, da für jede Probe ein räumlich begrenzter Amplifikationsbereich 24 vorgesehen ist und die Amplifikationsbereiche 24 voneinander getrennt sind.

Anschließend wird der Träger 18 mit den daran gebundenen Negativ-Kopien 7b', 7c', 7d', 7e' von dem Sequenzierer-Chip 14 entfernt und enthält eine Negativ-Kopie des Arrays der Nukleotide 7b, 7c, 7d, 7e in den Kavitäten 15 des Sequenzierer-Chips 14. Der Träger 18 mit den Negativ-Kopien 7b', 7c', 7d', 7e' kann einem weiteren Amplifikationsschritt unterzogen werden, um wiederum Positiv-Kopien 7b, 7c, 7d, 7e zu erhalten, die durch Verlängerung der Primer 22 erhalten werden. Ein solches Vorgehen ist im nachfolgenden Beispiel b) gezeigt.

Die Partikel 10 mit den Oligonukleotiden 7b, 7c, 7d, 7e verbleiben in den Kavitäten 15, so dass dieser wieder als primäres Array für einen erneuten Kopiervorgang mit einem neuen Träger dienen kann. Somit kann eine quasi beliebige Anzahl von Kopien erstellt werden.

Um nach dem Kopiervorgang das primäre Array (den Sequenzierer-Chip 14) wieder für einen weiteren Kopierzyklus vorzubereiten, kann das Amplifikationsmittel in den Kavitäten, beispielsweise der PCR-Mix, ersetzt oder entfernt werden.

### Ausführungsbeispiel b) (Fig. 5)

Die Beads 10 tragen jeweils unterschiedliche Oligonukleotide, jedoch mit denselben End-Sequenzen. Gezeigt ist der Prozess anhand der Oligonukleotids 7b. Die Kavitäten werden mit PCR-Mix, enthaltend löslichen Primer 30 befüllt (b) und mit einem Deckel 18 (zweiter Träger) verschlossen (c), der ein Adaptermolekül 22 (fixierter Primer) trägt. Die Enzyme des PCR-Mixes erzeugen in Schritt (d) zunächst eine Negativkopie 7b' des Oligonukleotids 7b auf dem Bead, welche sich durch Erhitzen löst (e). Durch einen Abkühlprozess (f) bindet die die Negativkopie 7b' an die Bindungsadapter 22 des Deckels und die Enzyme erzeugen dort in Schritt (g) eine fest gebundene Negativkopie 7b der Negativkopie, also eine Positivkopie 7b des originalen Oligonukleotids 7b. Am Ende des Prozesses wird der Deckel 18 abgenommen, auf dem sich nun eine Kopie (h) des Arrays (i) befindet. Die nicht kovalent gebundenen Negativkopien 7b', 7c', 7d', 7e' können in einem Waschschritt (nicht gezeigt) entfernt werden. Danach die Kopie des Arrays auf dem Träger 18 für Target-Bindungsversuche verwendet werden.

Die Vorlage (i) wird dabei nicht verbraucht, kann gewaschen und erneut mit einem PCR-Mix befüllt und wiederverwendet werden, z.B. zur Wiederholung des Prozesses und Herstellung weiterer Kopien.

## Patentansprüche

1. Verfahren zur Identifizierung von Aptameren, umfassend
- das in Kontakt bringen eines Gemisches von Oligonukleotiden mit einer Aptamer-Zielstruktur, und Binden zumindest eines Teils der Oligonukleotide an die Zielstruktur,
- das Abtrennen der Oligonukleotide, die an die Aptamer-Zielstruktur gebunden haben, von der Aptamer-Zielstruktur und von nicht an die Aptamer-Zielstruktur angebundenen Oligonukleotiden,
- das räumlich getrennte Amplifizieren aller Oligonukleotide, die an die Aptamer-Zielstruktur gebunden haben, und das Erzeugen räumlich getrennter Amplifikate für jedes Oligonukleotid, wobei jedes Amplifikat vorwiegend eine Sorte Oligonukleotide enthält,
- das Vergeben einer spezifischen Ortskennzeichnung an jedes der räumlich getrennten Amplifikate, so dass jedes der gekennzeichneten Amplifikate anhand seiner spezifischen Ortskennzeichnung eindeutig identifizierbar ist,
- das Sequenzieren von Oligonukleotiden der gekennzeichneten Amplifikate und die Zuordnung der für das Amplifikat spezifischen Ortskennzeichnung zu der Sequenz der Sorte Oligonukleotide in dem Amplifikat,
- Herstellen eines Arrays der Amplifikate, wobei die amplifizierten Oligonukleotide auf mindestens einen ersten Träger aufgebracht werden, so dass das Array einzelne Spots mit einheitlichen Sequenzen für jedes selektierte Oligonukleotid umfasst,
- die Analyse der Bindungseigenschaften aller Sorten von auf dem Array der Amplifikate angeordneten Oligonukleotiden an die Aptamer-Zielstruktur, und die Zuordnung der analysierten Bindungseigenschaften zu den spezifischen Ortskennzeichnungen der Amplifikate und den Sequenzen der entsprechenden Sorten von Oligonukleotiden,
- wobei zur Analyse der Bindungseigenschaften das Array der Amplifikate mit der Aptamer-Zielstruktur in Kontakt gebracht wird.

2. Verfahren nach Anspruch 1, bei dem das räumlich getrennte Amplifizieren ein Amplifizieren in einer Emulsion, ein digitales Amplifizieren oder ein Amplifizieren an einer festen Phase ist und/oder
bei dem die spezifische Ortskennzeichnung eine optisch, spektroskopisch, oder radioaktiv detektierbare Kennzeichnung ist.

3. Verfahren nach einem der vorangehenden Ansprüche, umfassend das Anbinden der Oligonukleotide vor, während oder nach dem räumlich getrennten Amplifizieren an Festphasenpartikel, wobei Festphasenpartikel erhalten werden, an die jeweils nur ein Amplifikat mit einer Sorte Oligonukleotide angebunden ist oder
umfassend das Anbinden der
Oligonukleotide vor, während oder nach dem räumlich getrennten Amplifizieren an Festphasenpartikel, wobei Festphasenpartikel erhalten werden, an die jeweils nur ein Amplifikat mit einer Sorte Oligonukleotide angebunden ist zusätzlich umfassend
- das in Kontakt bringen der Festphasenpartikel, an die jeweils ein Amplifikat mit einer Sorte Oligonukleotide angebunden ist, mit Aptamer-Zielstruktur,
- die Feststellung eines positiven oder negativen Bindungsereignisses der Aptamer-Zielstruktur an Oligonukleotide des Amplifikats, das an ein Festphasenpartikel angebunden ist,
- die Selektion von Festphasenpartikeln, bei denen ein positives Bindungsereignis feststellbar ist.

4. Verfahren nach einem der Ansprüche 1-3, umfassend
- das Erzeugen einer Kopie oder eines Derivats des Arrays der Amplifikate an, auf oder in einem zweiten Träger, wobei jedem Amplifikat der Kopie oder des Derivats eine spezifische Ortskennzeichnung zugewiesen wird,
- das in Kontakt bringen der Kopie oder des Derivats des Arrays mit der Aptamer-Zielstruktur zur Analyse der Bindungseigenschaften.

5. Verfahren nach Anspruch 4, wobei der zweite Träger einen Bindungsadapter für Oligonukleotide der Amplifikate oder Bindungseigenschaften für Oligonukleotide der Amplifikate aufweist, und die Kopie erzeugt wird, indem Oligonukleotide aus den Amplifikaten mittels des Bindungsadapters oder der Bindungseigenschaften an den Träger gebunden werden.

6. Verfahren nach Anspruch 5 umfassend
- die Bereitstellung zumindest eines räumlich begrenzten Amplifikationsmittelbereichs für jedes Oligonukleotid, der von den Amplifikationsmittelbereichen der anderen Oligonukleotide getrennt ist, wobei eine mit dem Bindungsadapter oder Bindungseigenschaften versehene Oberfläche des zweiten Trägers an die Amplifikationsmittelbereiche angrenzt,
- das Amplifizieren der Oligonukleotide mittels Amplifikationsmitteln in den Amplifikationsmittelbereichen zum Erzeugen von Amplifikaten der Oligonukleotide,
- das Binden von Einzelsträngen oder Derivaten der Amplifikate an den zweiten Träger mittels des Bindungsadapters oder der Bindungseigenschaften, so dass eine räumliche Anordnung der Einzelstränge auf dem zweiten Träger der räumlichen Anordnung der Amplifikate in dem Array entspricht, aus dem die Einzelstränge stammen; und
- das Entfernen des zweiten Trägers mit den angebundenen Einzelsträngen von dem Array.

7. Verfahren nach Anspruch 6, bei dem das Binden der Kopien oder Derivate an den zweiten Träger gleichzeitig mit dem Amplifizieren erfolgt.

8. Verfahren nach Anspruch 6 oder 7 bei dem die räumlich begrenzten Amplifikationsmittelbereiche zumindest teilweise durch Mikro- oder Nanostrukturen in dem ersten, das Array tragenden Träger oder in dem zweiten, die Kopie oder das Derivat tragenden Träger definiert sind.

9. Verfahren nach Anspruch 6 oder 7 bei dem die räumlich begrenzten Amplifikationsmittelbereiche zumindest teilweise durch Phasengrenzen zwischen zwei Flüssigkeiten, einer Flüssigkeit und einem Gas oder einer physikalischen Grenze getrennt sind.

10. Verfahren nach Anspruch 8,
bei dem das Erzeugen zumindest eines räumlich begrenzten Amplifikationsmittelbereichs ein Bereitstellen des zweiten Trägers mit zumindest einer jedem Oligonukleotid zugeordneten Ausnehmung, in der der Bindungsadapter angeordnet ist, ein Einbringen des Amplifikationsmittels in die Ausnehmungen und ein Verschließen der Ausnehmungen mittels des ersten Trägers, so dass die Oligonukleotide dem Amplifikationsmittelbereich ausgesetzt sind, aufweist oder
bei dem das Erzeugen zumindest eines räumlich begrenzten Amplifikationsmittelbereichs für jedes Oligonukleotid ein Bereitstellen der Oligonukleotide in jeweils zugeordneten separaten Ausnehmungen in dem ersten, das Array tragenden Träger, ein Einbringen des Amplifikationsmittels in die Ausnehmungen und ein Verschließen der Ausnehmungen durch den zweiten Träger aufweist.

11. Verfahren nach mindestens einem der vorherigen Ansprüche, umfassend die Rückgewinnung einer oder
mehrerer Sorten von Oligonukleotiden durch Lösen der Oligonukleotide von dem ersten Träger, dem zweiten Träger und/oder von Kopien dieser Träger oder
umfassend die Herstellung weiterer Oligonukleotide durch Amplifizieren von Oligonukleotiden, die auf dem ersten Träger, dem zweiten Träger und/oder auf Kopien dieser Träger gebunden sind.

12. Verfahren zur Identifizierung von Aptamer-Bindungspaaren, die an verschiedene Stellen einer Aptamer-Zielstruktur binden, umfassend die Schritte des Verfahrens nach einem der Ansprüche 1-8, und weiterhin umfassend:
a) das in Kontakt bringen des Arrays der Amplifikate oder der Kopie des Arrays der Amplifikate, wie aus einem Verfahren nach einem der Ansprüche 1-9 erhalten, mit der Aptamer-Zielstruktur und Bindung der Aptamer-Zielstruktur an Oligonukleotide, die in Amplifikaten des Arrays/der Kopie des Arrays enthalten sind,
b) das in Kontakt bringen des in a) erhaltenen Arrays oder der Kopie des Arrays mit einem Gemisch von Oligonukleotiden und die Bindung zumindest eines Teils der Oligonukleotide an die Aptamer-Zielstruktur, die bereits auf dem Array gebunden ist,
c) wobei sich die jeweiligen Bindungsstellen der bindenden Oligonukleotide aus dem Gemisch von den Bindungsstellen der auf dem Array befindlichen Oligonukleotide, die an die jeweils selbe Zielstruktur binden, unterscheiden,
d) die Elution der Oligonukleotide, die in b) nicht an die Aptamer-Zielstruktur gebunden haben,
e) das Entfernung der in b) gebundenen Oligonukleotide von der Aptamer-Zielstruktur
f) die Sequenzierung der in d) erhaltenen Oligonukleotide, und die Herstellung oder Isolierung der Oligonukleotide in sortenreiner Form,
g) das in Kontakt bringen eines Arrays der Amplifikate oder der Kopie des Arrays der Amplifikate wie aus einem Verfahren nach einem der Ansprüche 1-8 erhalten, mit der Aptamer-Zielstruktur und Bindung der Aptamer-Zielstruktur an Oligonukleotide, die in Amplifikaten des Arrays/der Kopie des Arrays enthalten sind,
h) das in Kontakt bringen des in f) erhaltenen Arrays oder der Kopie des Arrays mit einem sortenreinen Oligonukleotid aus e) und die Bindung des Oligonukleotids an eine oder mehrere Aptamer-Zielstrukturen, die bereits auf dem Array gebunden ist/sind,
i) die Analyse, an welche Aptamer-Zielstruktur(en) auf dem Array das sortenreine Oligonukleotid gebunden hat und die Zuordnung dieser Zielstruktur zu dem Oligonukleotid des Arrays, an das die Zielstruktur gebunden hat und dessen spezifischer Ortskennzeichnung in dem Array,
j) optional die ein- oder mehrfache Wiederholung der Schritte g) und h).

## Claims

1. A method for identification of aptamers, comprising
- contacting a mixture of oligonucleotides with an aptamer-target structure and binding at least a portion of the oligonucleotides to the target structure,
- separating the oligonucleotides having bound to the aptamer-target structure from the aptamer-target structure and from oligonucleotides not having bound to the aptamer-target structure,
- amplifying in a spatially separated manner all oligonucleotides bound to the aptamer-target structure and generating spatially separated amplification products for each oligonucleotide, wherein each amplification product contains primarily one type of oligonucleotide,
- assigning a specific position label to each of the spatially separated amplification products, so that each of the labeled amplification products is unequivocally identifiable based on its specific position label,
- sequencing oligonucleotides of the identified amplification products and assigning the position label specific for the amplification product
to the sequence of the type of
oligonucleotides in the amplification product
- producing an array of the amplification products, wherein the amplified oligonucleotides are applied to at least one first carrier so that the array comprises individual spots with uniform sequences for each selected oligonucleotide,
- analyzing the binding characteristics of all types of oligonucleotides arranged on the array of amplification products to the aptamer-target structure, and assigning the binding characteristics analyzed to the specific position labels of the amplification products and the sequences of the corresponding types of oligonucleotides,
- wherein for analyzing the binding characteristics, the array of the amplification products is brought into contact with the aptamer-target structure.

2. The method according to claim 1, in which the spatially separated amplification is amplification in an emulsion, digital amplification or amplification on a solid phase and/or in which the specific position label is an optically, spectroscopically or radioactively detectable label.

3. The method according to one of the preceding claims, comprising the binding of the oligonucleotides before, during or after the spatially separated amplification to solid-phase particles, to which in each case only one amplification product with one type of oligonucleotides is bound or comprising the binding of the
oligonucleotides before, during or after the spatially separated amplification to solid-phase particles, wherein solid-phase particles are obtained to which in each case only one amplification product with one type of oligonucleotide is bound, additionally comprising
- contacting the solid-phase particles, to which in each case an amplification product with one type of oligonucleotides is bound, with an aptamer-target structure,
- detecting a positive or negative binding event of the aptamer-target structure to oligonucleotides of the amplification product bound to a solid-phase particle,
- selecting solid-phase particles in which a positive binding event can be detected.

4. The method according to one of claims 1-3, comprising
- creating a copy or a derivative of the array of the amplification products at, on or in a second carrier, wherein each amplification product of the copy or the derivative is assigned to a specific position label,
- contacting the copy or the derivative of the array with the aptamer-target structure for analysis of the binding properties.

5. The method according to claim 4, wherein the second carrier has a binding adapter for oligonucleotides of the amplification products or binding characteristics for oligonucleotides of the amplification products, and the copy is produced in that oligonucleotides from the amplification products are bound to the carrier by means of the binding adapter or the binding characteristics.

6. The method according to claim 5 comprising
- supplying at least one spatially delimited amplification agent region for each oligonucleotide, which is separated from the amplification agent regions of the other oligonucleotides, wherein a surface of the second carrier provided with the binding adapter or binding characteristics is adjacent to the amplification agent region,
- amplifying the oligonucleotides using amplification agents in the amplification agent regions to create amplification products of the oligonucleotides,
- binding individual strands or derivatives of the amplification agents to the second carrier using the binding adapter or the binding characteristics, so that a spatial arrangement of the individual strands on the second carrier corresponds to the spatial arrangement of the amplification agents in the array from which the individual strands originate, and
- removing the second carrier with the individual strands bound to it from the array.

7. The method according to claim 6, in which the binding of the copies or derivatives to the second carrier takes place simultaneously with the amplification.

8. The method according to claims 6 or 7, in which the spatially delimited amplification agent regions are defined at least partially by micro- or nanostructures in the first carrier, bearing the array, or in the second carrier, bearing the copy or the derivative.

9. The method according to claim 6 or 7, in which the spatially delimited amplification agent regions are separated at least partially by interfaces between two liquids, a liquid and a gas or a physical boundary.

10. The method according to claim 8,
in which the creation of at least one spatially delimited amplification agent region involves supplying the second carrier with at least one recess assigned to each oligonucleotide, in which the binding adapter is disposed, introducing the amplification agent into the recesses, and closing the recesses using the first carrier, so that oligonucleotides are exposed to the amplification agent region or
in which the creation of at least one spatially delimited amplification agent region for each oligonucleotide involves supplying the oligonucleotides in the respectively assigned separate recesses in the first carrier bearing the array, introducing the amplification agent into the recesses, and closing the recesses using the second carrier.

11. The method according to at least one of the preceding claims, comprising the recovery of one or
more types of oligonucleotides by freeing the oligonucleotides from of the first carrier, the second carrier and/or from copies of these carriers or
comprising the production of additional nucleotides by amplification of oligonucleotides bound to the first carrier, the second carrier and/or copies of these carriers.

12. The method for identification of aptamer-bond pairs that bind at different sites on an aptamer-target structure, comprising the steps of the method according to any one of claims 1-8, and also comprising:
a) contacting the array of the amplification product or the copy of the array of the amplification product, as obtained from any one of claims 1-9, with the aptamer-target structure and binding aptamer-target structure to oligonucleotides contained in amplification products of the array/copy of the array,
b) contacting the array or copy of the array obtained in a) with a mixture of oligonucleotides and binding at least a portion of the oligonucleotides to the aptamer-target structure that is already bound to the array,
c) wherein the respective binding sites of the binding oligonucleotides from the mixture differ from the binding sites of the oligonucleotides located on the array, which bind to the same target structure in each case,
d) eluting the oligonucleotides that have not bound to the aptamer-target structure in b),
e) removing the oligonucleotides bound in b) from the aptamer-target structure
f) sequencing the oligonucleotides obtained in d) and production or isolation of the oligonucleotides in single-type form,
g) contacting an array of the amplification products or the copy of the array of the amplification products as obtained from a method according to claims 1-8 with the aptamer-target structure and binding the aptamer-target structure to oligonucleotides contained in amplification products of the array/the copy of the array.
h) contacting the array or copy of the array obtained in f) with a single-type oligonucleotide from e) and binding the oligonucleotide to one or more aptamer-target structures already bound to the array,
i) analyzing to which aptamer-target structure(s) on the array the single-type oligonucleotide has bound and assigning this target structure to the oligonucleotide of the array to which the target structure has bound and its specific position label in the array,
j) optionally repeating steps g) and h) one or more times.

## Revendications

1. Procédé pour identifier des aptamères, comprenant
- la mise en contact d'un mélange d'oligonucléotides avec une structure cible d'aptamère, et la liaison d'au moins une partie des oligonucléotides sur la structure cible,
- la séparation des oligonucléotides qui sont liés à la structure cible d'aptamère de la structure cible d'aptamère et des oligonucléotides non liés à la structure cible d'aptamère,
- l'amplification séparée spatialement de tous les oligonucléotides qui sont liés à la structure cible d'aptamère, et la génération de produits d'amplification séparés spatialement pour chaque oligonucléotide, dans lequel chaque produit d'amplification contient principalement une sorte d'oligonucléotides,
- l'attribution d'une identification de position spécifique à chacun des produits d'amplification séparés spatialement, de sorte que chacun des produits d'amplifications marqué soit identifiable clairement à partir de son identification de position spécifique,
- le séquençage d'oligonucléotides des produits d'amplification marqués et l'affectation de l'identification de position spécifique, pour le produit d'amplification, à la séquence de la sorte d'oligonucléotides dans le produit d'amplification,
- la production d'une matrice de produits d'amplification, dans lequel les oligonucléotides amplifiés sont appliqués sur au moins un premier support de sorte que la matrice comprenne des spots individuels avec des séquences unitaires pour chaque oligonucléotide sélectionné,
- l'analyse des propriétés de liaison de toutes les sortes d'oligonucléotides disposés sur la matrice des produits d'amplification à la structure cible d'aptamère, et l'affectation des propriétés de liaison analysées aux identifications de position spécifiques des produits d'amplification et aux séquences des sortes correspondantes d'oligonucléotides,
- dans lequel pour l'analyse des propriétés de liaison, la matrice des produits d'amplification est mise en contact avec la structure cible d'aptamère.

2. Procédé selon la revendication 1, dans lequel l'amplification séparée spatialement est une amplification dans une émulsion, une amplification numérique ou une amplification sur une phase solide et/ou
dans lequel l'identification de position spécifique est une identification pouvant être détectée de façon optique, spectroscopique ou radioactive.

3. Procédé selon l'une des revendications précédentes, comprenant la liaison des oligonucléotides avant, pendant ou après l'amplification séparée spatialement sur des particules en phase solide, dans lequel on obtient des particules en phase solide sur lesquelles respectivement seul un produit d'amplification est lié à une sorte d'oligonucléotide ou comprenant la liaison des oligonucléotides avant, pendant ou après l'amplification séparée spatialement sur des particules en phase solide, dans lequel on obtient des particules en phase solides sur lesquelles respectivement seul un produit d'amplification est lié à une sorte d'oligonucléotide comprenant en outre
- la mise en contact des particules en phase solide sur lesquelles respectivement un produit d'amplification est lié à une sorte d'oligonucléotide, avec une structure cible d'aptamère,
- la détermination d'un événement de liaison positif ou négatif de la structure cible d'aptamère aux oligonucléotides du produit d'amplification qui est lié à une particule en phase solide,
- la sélection de particules en phase solide, dans lesquelles un événement de liaison positif peut être déterminé.

4. Procédé selon l'une des revendications 1 à 3, comprenant
- la génération d'une copie ou d'un dérivé de la matrice de produits d'amplification, sur ou dans un deuxième support, dans lequel chaque produit d'amplification de la copie ou du dérivé est affecté à une identification de position spécifique,
- la mise en contact de la copie ou du dérivé de la matrice avec la structure cible d'aptamère pour l'analyse des propriétés de liaison.

5. Procédé selon la revendication 4, dans lequel le deuxième support présente un adaptateur de liaison pour des oligonucléotides des produits d'amplification ou des propriétés de liaison pour des oligonucléotides des produits d'amplification, et la copie est générée, en liant des oligonucléotides issus des produits d'amplification au moyen de l'adaptateur de liaison ou des propriétés de liaison sur un support.

6. Procédé selon la revendication 5, comprenant
- la mise à disposition d'au moins un milieu d'amplification limité spatialement pour chaque oligonucléotide, dans lequel une surface dotée de l'adaptateur de liaison ou des propriétés de liaison du deuxième support est adjacente aux milieux d'amplification,
- l'amplification des oligonucléotides au moyen d'agents d'amplification dans les milieux d'amplification pour générer des produits d'amplification des oligonucléotides,
- la liaison de brins simples ou de dérivés des produits d'amplification sur le deuxième support au moyen de l'adaptateur de liaison ou des propriétés de liaison, de sorte qu'une disposition spatiale des brins simples sur le deuxième support corresponde à la disposition spatiale des produits d'amplification dans la matrice à partir de laquelle proviennent les brins simples ; et
- le retrait du deuxième support avec les brins simples liés de la matrice.

7. Procédé selon la revendication 6, dans lequel la liaison des copies ou des dérivés au deuxième support s'effectue en même temps que l'amplification.

8. Procédé selon la revendication 6 ou 7 dans lequel les milieux d'amplification limités spatialement sont définis au moins partiellement par des micro- ou nanostructures dans le premier support portant la matrice ou dans le deuxième support portant la copie ou le dérivé.

9. Procédé selon la revendication 6 ou 7, dans lequel les milieux d'amplification spatialement limités sont séparés au moins par des limites de phases entre deux liquides, entre un liquide et un gaz ou une barrière physique.

10. Procédé selon la revendication 8,
dans lequel la génération d'au moins un milieu d'amplification limité spatialement présente une mise à disposition du deuxième support avec au moins un évidement affecté à chaque oligonucléotide, dans lequel l'adaptateur de liaison est disposé, un apport du milieu d'amplification dans les évidements et une fermeture des évidements au moyen du premier support, de sorte que les oligonucléotides soient exposés au milieu d'amplification, ou
dans lequel la génération d'au moins un milieu d'amplification limité spatialement pour chaque oligonucléotide présente une mise à disposition des oligonucléotides dans des évidements séparés affectés respectivement dans le premier support portant la matrice, un apport du milieu d'amplification dans les évidements et une fermeture des évidements par le deuxième support.

11. Procédé selon au moins l'une des revendications précédentes, comprenant la récupération d'une ou plusieurs sortes d'oligonucléotides par détachement des oligonucléotides du premier support, du deuxième support et/ou de copies de ces supports ou comprenant la production d'autres oligonucléotides par amplification d'oligonucléotides qui sont liés au premier support, au deuxième support et/ou à des copies de ces supports.

12. Procédé d'identification de paires de liaison d'aptamères, qui se lient à différents endroits d'une structure cible d'aptamère, comprenant les étapes du procédé selon l'une des revendications 1 à 8, et comprenant en outre :
a) la mise en contact de la matrice des produits d'amplification ou de la copie de la matrice des produits d'amplification, tels qu'obtenus dans un procédé selon l'une des revendications 1 à 9, avec la structure cible d'aptamère et la liaison de la structure cible d'aptamère aux oligonucléotides qui sont contenus dans les produits d'amplification de la matrice / de la copie de la matrice,
b) la mise en contact de la matrice obtenue au point a) ou de la copie de la matrice avec un mélange d'oligonucléotides et la liaison d'au moins une partie des oligonucléotides à la structure cible d'aptamère, qui est déjà liée à la matrice,
c) dans lequel les sites de liaison respectifs des oligonucléotides liants sont distincts du mélange des sites de liaison des oligonucléotides se trouvant sur la matrice qui se lient respectivement à la même structure cible,
d) l'élution des oligonucléotides qui ne se sont pas liés à la structure cible d'aptamère au point b),
e) le retrait des oligonucléotides liés au point b) de la structure cible d'aptamère,
f) le séquençage des oligonucléotides obtenus au point d) et la production ou l'isolement des oligonucléotides dans une forme présentant une sorte pure,
g) la mise en contact d'une matrice des produits d'amplification ou de la copie de la matrice des produits d'amplification comme dans un procédé selon l'une des revendications 1 à 8, avec la structure cible d'aptamère et la liaison de la structure cible d'aptamère aux oligonucléotides qui sont contenus dans les produits d'amplification de la matrice / de la copie de la matrice,
h) la mise en contact de la matrice ou de la copie de la matrice obtenue au point f) avec un oligonucléotide présentant une sorte pure du point e) et la liaison de l'oligonucléotide à une ou plusieurs structure (s) cible(s) d'aptamère qui est/sont déjà liée(s) sur la matrice,
i) l'analyse déterminant sur quelle(s) structure(s) cible(s) d'aptamère sur la matrice, l'oligonucléotide présentant une sorte pure s'est lié et l'affectation de cette structure cible à l'oligonucléotide de la matrice, auquel la structure cible s'est liée et son identification de position spécifique dans la matrice,
j) éventuellement la répétition une ou plusieurs fois des étapes g) et h).
